# EUROPEAN PATENT SPECIFICATION

(11) **EP 2 097 428 B1**
(45) Date of publication and mention of the grant of the patent: **28.02.2018**
(21) Application number: 07868639.1
(22) Date of filing: 31.10.2007
(51) Int. Cl.: C07F 9/12, C07F 9/46, C07F 9/24, C07F 9/40

(54) **BUTYRYLCHOLINESTERASE INHIBITORS AND THEIR USE IN THE TREATMENT OF NEURODEGENERATIVE DISEASES**
PSEUDOCHOLINESTERASE-HEMMER UND IHRE VERWENDUNG BEI DER BEHANDLUNG NEURODEGENERATIVER ERKRANKUNGEN
IINHIBITEURS DE BUTYRYLCHOLINESTERASE ET LEUR UTILISATION DANS LE TRAITEMENT DES MALADIES NEURODÉGÉNÉRATIVES

(30) Priority: 31.10.2006 US 863764 P
(43) Date of publication of application: 09.09.2009
(73) Proprietor: Mgp Biotechnologies, Inc., Irvine CA 92614 (US)
(72) Inventor: ACEY, Roger A., Long Beach, CA 90808 (US); NAKAYAMA, Kensaku, Tustin, CA 92780 (US)
(74) Representative: Pons
(86) International application number: PCT/US2007/083262
(87) International publication number: WO 2008/055249

(56) References cited:
- WO-A-99/52516
- WO-A-99/58555
- US-A- 2 944 937
- PRUETT S B ET AL: "A comparative study of inhibition of acetylcholinesterase, trypsin, neuropathy target esterase, and spleen cell activation by structurally related organophosphorus compounds" JOURNAL OF BIOCHEMICAL TOXICOLOGY, vol. 9, no. 6, December 1994 (1994-12), pages 319-327, XP009099645 ISSN: 0887-2082
- MAGER P P ET AL: "Structure-activity relationships applied to phenyl diethyl phosphate pesticides" PHARMAZIE, vol. 37, no. 10, 1982, pages 729-730, XP001538137 ISSN: 0031-7144
- NAGAMATSU T ET AL: "New phosphorylating agents for general synthesis of mixed phosphate esters" TETRAHEDRON LETTERS, vol. 28, no. 21, 1987, pages 2375-2378, XP009099643 ISSN: 0040-4039
- BATRA B S ET AL: "Phase transfer catalysed phosphorylation of phenols: Phosphate esters" INDIAN JOURNAL OF CHEMISTRY - SECTION B ORGANIC CHEMISTRY INCLUDING MEDICINAL CHEMISTRY, vol. 30, no. 1, 1991, pages 57-58, XP009099649 ISSN: 0019-5103
- SIDDALL T H ET AL: "Conformation of Organophosphorus Compounds. II. Proton Magnetic Resonance Studies of Some Phosphites, Phosphonites, Phosphates, Phosphonates and Additional Phosphinates" JOURNAL OF THE AMERICAN CHEMICAL SOCIETY., vol. 84, no. 18, 20 September 1962 (1962-09-20), pages 3467-3473, XP007904652 ISSN: 0002-7863
- JENTZSCH R ET AL: "Organophosphorverbindungen. VI. Quantitative Untersuchungen über das Alkylierungsvermögen kernsubstituierter O,O-Dimethyl-O-phenyl-phosphate und thiophosphate" JOURNAL FÜR PRAKTISCHE CHEMIE, vol. 317, no. 5, 1975, pages 721-732, XP009099637
- THAU-ALEXANDROWICZ G ET AL: "Viscosity study of certain alkyl phosphates and their aqueous mixtures" JOURNAL OF CHEMICAL AND ENGINEERING DATA, vol. 17, no. 3, 1972, pages 339-341, XP009099629
- DE ROOS A M ET AL: "The preparation of some dialkyl p-nitrophenyl phosphates" RECUEIL DES TRAVAUX CHIMIQUES DES PAYS-BAS, vol. 77, 1958, pages 946-950, XP009099660 ISSN: 0165-0513
- VAN HOOIDONK C ET AL: "On the reactivity of organophosphorus compounds. Part III. Application of the Hammett relation to the rates of alkaline hydrolysis of a number of diethyl substituted phenyl phosphates" RECUEIL DES TRAVAUX CHIMIQUES DES PAYS-BAS, vol. 86, 1967, pages 449-457, XP009099668 ISSN: 0165-0513
- BUDNIKOVA Y G ET AL: "Dimer formation in the reaction of aryl halides catalysed by nickel complexes" BULLETIN OF THE RUSSIAN ACADEMY OF SCIENCES, DIVISION OF CHEMICAL SCIENCE., vol. 41, no. 7.2, 1992, pages 1299-1300, XP009099663 ISSN: 1063-5211

## Description

### FIELD OF THE INVENTION

The present disclosure relates to inhibitors of butyrylcholinesterase.

### BACKGROUND OF THE INVENTION

Neurodegenerative diseases result from deterioration of neurons which over time will lead to neurodegeneration and disabilities.

It is known that reduction in levels of acetylcholine parallels the severity of a neurodegenerative disorder such as Alzheimer's disease (AD). In addition, progression of AD occurs concomitantly with changes in cholinesterase activity, i.e., acetylcholinesterase activity (AcChase) decreases while butyrylcholinesterase (BuChase) activity increases. Since both enzymes hydrolyze acetylcholine, the treatment for AD is based on the assumption that inhibiting the activity of these enzymes, in particular butyrylcholinesterase, will increase the level of acetylcholine. Unfortunately, currently utilized cholinesterase inhibitors are non-specific and show adverse peripheral effects.

Several neurodegenerative disorders are also associated with the formation of beta-amyloid plaques. They seem to be formed in the brain many years before the clinical signs of the disorder, e.g. AD, are detectable. Beta-amyloid plaque formation is associated with BuChase activity. Therefore, BuChase inhibitors can have a significant effect on preventing or retarding the formation of beta-amyloid plaques.

WO 99/58555 discloses water- soluble prodrugs and pharmaceutical preparations of propofol prodrugs, which are formulated to deliver the beneficial effects of propofol without harmful side effects arising from earlier formulations. The water-soluble prodrugs of propofol, such as those disclosed in the above-referenced PCT application, are useful in methods of treating or preventing migraine in subjects suffering from or prone to this condition.

Certian organophosphorous compounds have been shown to bind to and inactivate several target molecules other than acetylcholinesterase (see for example, PRUETT S BET AL: "A comparative study of inhibition of acetylcholinesterase, trypsin, neuropathy target esterase, and spleen cell activation by structurally related organophosphorus compounds" JOURNAL OF BIOCHEMICAL TOXICOLOGY, vol. 9, no. 6, December 1994, pages 319-327).

WO 99/52516 shows that cognitive enhancement noted in mammalian subjects upon consumption of certain organophosphorous substances is due, not to inhibition of acetylcholinesterase as is conventionally suggested, but due to inhibition of a different enzyme, acylpeptide hydrolase.

The compounds described by MAGER P PETAL (see "structure-activity relationships applied to phenyl diethyl phosphate pesticides" PHARMAZIE, val. 37, no. 10, 1982, pages 729-730 04) have been tested for their inhibitory effect on acetylcholinesterase.

In addition, there is a general need for BuChase specific inhibitors. These compounds may be used in various biochemical, pharmacological, and cell biology applications to study the role of BuChase in normal cell growth and development, e.g., stem cell differentiation. Therefore, there is an unmet need for specific inhibitors of butyrylcholinesterase.

### SUMMARY OF THE INVENTION

One aspect of the disclosure relates to inhibitors of butyrylcholinesterase for the treatment of neurodegenerative diseases. These inhibitors have the following general formulas: 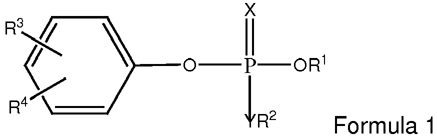 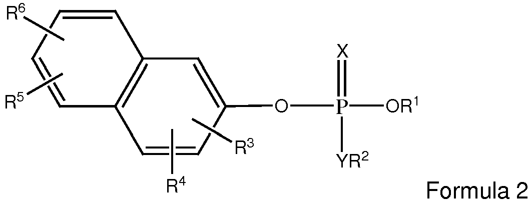 wherein
X is O or S;
Y is O or N;
R¹ and R² can be the same or different and are independently selected from the group consisting of H, C₁₋₆ alkyl, C₁₋₆ alkenyl, and unsubstituted or substituted phenyl;
R³ to R⁶ can be the same or different and are independently selected from the group consisting of H, methyl, methoxy, and at least one electron withdrawing group;
or a pharmaceutically acceptable salt thereof.

Another aspect of the disclosure relates to pharmaceutical compositions containing a pharmaceutical carrier and a therapeutically effective amount of these inhibitors of butyrylcholinesterase.

Another aspect of the disclosure relates to the use of these inhibitors of butyrylcholinesterase in the mannufacture of a medicament for the treatment of a neurodegenerative disease.

### BRIEF DESCRIPTION OF THE DRAWINGS

Figure 1 depicts the effect of di-n-butyl 2-chlorophenyl phosphate (DB2CIPP) on acetylcholinesterase (AcChase) activity. AcChase was pre-incubated with either solvent or di-n-butyl 2-chlorophenyl phosphate, fractionated by native gel electrophoresis, and stained for enzyme activity.
Figures 2A and 2B depict phosphorylation of butyrylcholinesterase by di-ethyl 2-chlorophenyl phosphate. Butyrylcholinesterase was incubated with ¹⁴C-labeled diethyl 2-chloro phenyl phosphate, or solvent, then separated using native PAGE. A) Gel stained for enzyme activity. B) Autoradiography of the gel.
Figure 3 depicts the effect of DB2CIPP on the activity of trypsin.
Figure 4 depicts the effect of DB2CIPP on the activity of chymotrypsin.
Figure 5 depicts the effect of di-n-butyl 2-chlorophenyl phosphate on the activity of PKA and S6K2. Protein Kinase A (PKA) and S6K2 were incubated with the indicated concentrations of di-n-butyl 2-chlorophenyl phosphate and assayed as described previously. The reaction mixtures were separated by SDS-PAGE, and then stained for phosphoproteins with Pro-Q® Diamond.
Figure 6 depicts the effect of DB2CIPP on hexokinase activity.
Figure 7 depicts the lack of toxicity of di-n-butyl 2-chlorophenyl phosphate on porcine umbilical stem cells. Cells were cultured in Neurobasal Medium with increasing concentrations of di-n-butyl 2-chlorophenyl phosphate for 48 hours. Solvent acted as control. Each concentration had its own corresponding control group. Results are expressed as % mortality and are the mean of 3 replicates ± SD.
Figure 8 is a stick rendering which models the flexible docking of a phosphate with AcChase and BuChase.
Figure 9 is a stick rendering which models the flexible docking of a phosphonate with AcChase and BuChase.
Figure 10 is a stick rendering modeling the flexible docking of a phosphinate with AcChase and BuChase.
Figure 11 is a stick rendering modeling the flexible docking of a phosphoramidate with AcChase and BuChase.
Figure 12 shows a spectrum resulting from Matrix Assisted Laser Desorption/Ionization Time of Flight (MALDI-TOF) Mass Spectroscopy. BuChase was incubated with di-n-butyl-2-chlorophenyl phosphate. The excess inhibitor was removed and BuChase was digested with trypsin. The tryptic peptides were then analyzed by MALDI-TOF.

### DETAILED DESCRIPTION OF THE INVENTION

One aspect of the disclosure relates to inhibitors of butyrylcholinesterase (BuChase).

The butyrylcholinesterase inhibitors can be represented by a compound of Formula 1 or Formula 2 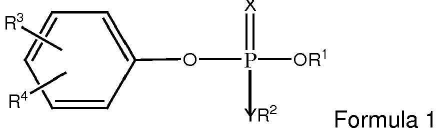 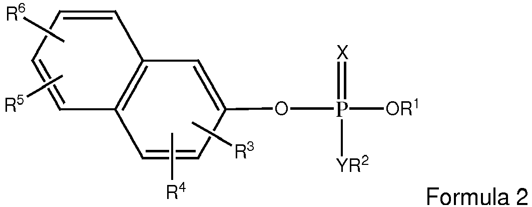 wherein
X is O or S;
Y is O or N;
R¹ and R² can be the same or different and are independently selected from the group consisting of H, C₁₋₆ alkyl, C₁₋₆ alkenyl, and unsubstituted or substituted phenyl; wherein the potential substituents of the substituted phenyl group are selected from F, Cl, Br, I, or NO₂;
R³ and R⁴ in formula I can be the same or different and are independently selected from the group consisting of H, methyl, methoxy, F, Cl, Br and I; and
R³ to R⁶ in formula II can be the same or different and are independently selected from the group consisting of H, methyl, methoxy, F, Cl, Br, I and NO₂;
or a pharmaceutically acceptable salt thereof.

Alternatively, when R¹ and/or R² is phenyl, the phenyl maybe substituted at least once wherein each substituent is independently selected from the group consisting of H, methyl, methoxy, F, Cl, Br, I and NO₂.

Alternatively, the C₁₋₆ alkyl is selected from the group consisting of methyl, ethyl, n-propyl, isopropyl, n-butyl, isobutyl, cyclohexyl and n-pentyl.

Exemplary compounds of Formulas 1 and 2 include but are not limited to: 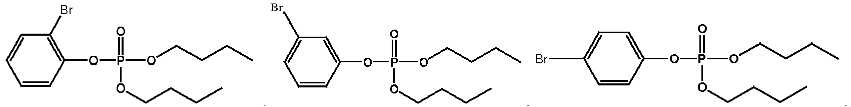 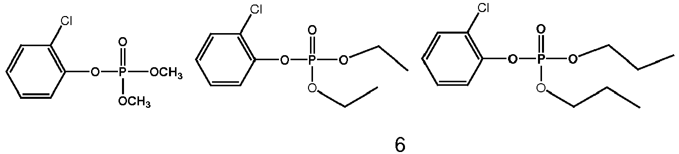 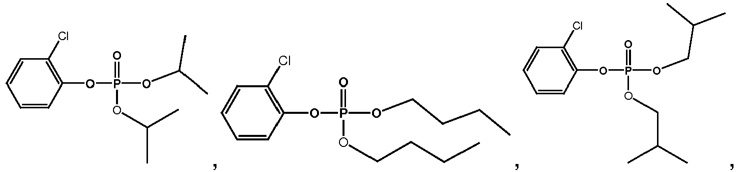 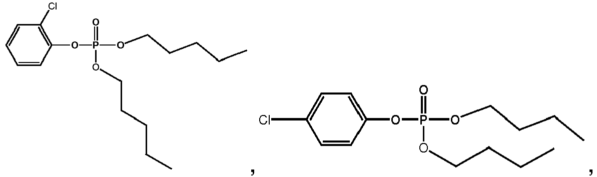 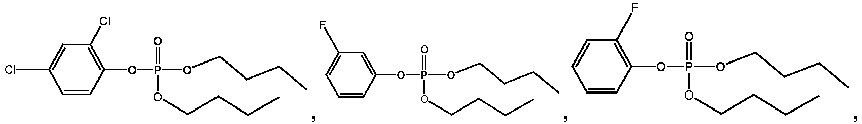 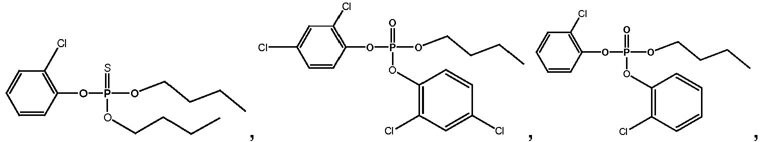 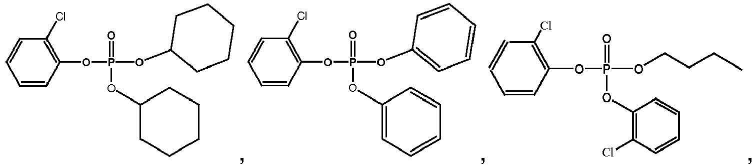 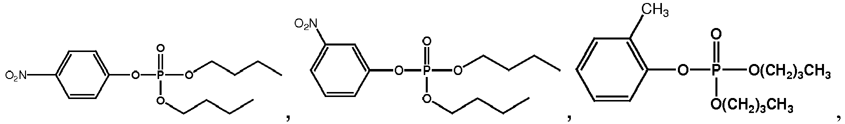 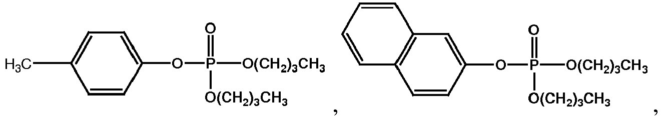 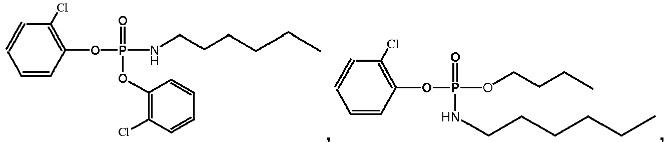 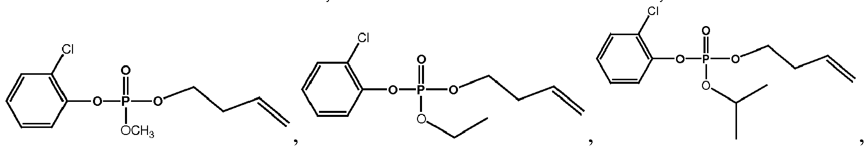 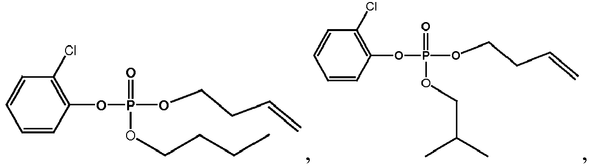 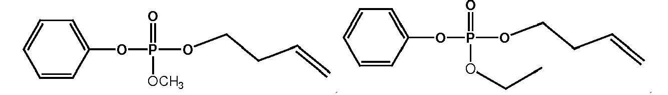 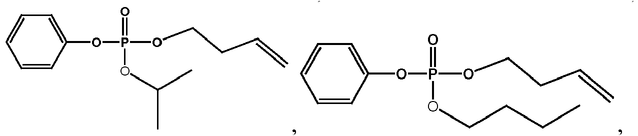 and

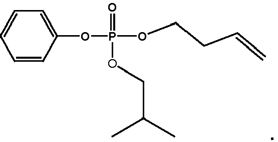

A "pharmaceutically acceptable salt" as used herein is any salt that retains at least some of the activity of the parent compound and without imparting any additional deleterious or untoward effects on the subject to which it is administered and in the context in which it is administered compared to the parent compound. A pharmaceutically acceptable salt also refers to any salt which may form *in vivo* as a result of administration of an acid, another salt, or a prodrug which is converted into an acid or salt.

Pharmaceutically acceptable salts of acidic functional groups may be derived from organic or inorganic bases. The salt may comprise a mono or polyvalent ion. Of particular interest are the inorganic ions, lithium, sodium, potassium, calcium, and magnesium. Organic salts may be made with amines, particularly ammonium salts such as mono-, di- and trialkyl amines or ethanol amines. Salts may also be formed with caffeine, tromethamine and similar molecules. Hydrochloric acid or some other pharmaceutically acceptable acid may form a salt with a compound that includes a basic group, such as an amine or a pyridine ring.

Another aspect of the present disclosure is drawn to therapeutic compositions comprising the presently disclosed compounds and a pharmaceutically acceptable carrier. The carrier may be any solid, semi-solid, or liquid material that acts as an excipient or vehicle for the active compound. The formulations may also include wetting agents, emulsifying agents, preserving agents, sweetening agents, bulking agents, coatings and/or flavoring agents. If used in an ophthalmic or infusion format, the formulation will usually contain one or more salts to adjust the osmotic pressure of the formulation.

The present disclosure provides for inhibitors of butyrylcholinesterase for use in the treatment of a condition or disease which is ameliorated by cholinesterase inhibition. Without wishing to be bound by theory, one of the ways cholinesterase inhibition can be helpful in the treatment of a neurodegenerative disease is by eliminating, reducing, or preventing the formation of beta amyloid plaques.

The diseases or conditions which can be treated by the presently disclosed inhibitors of butyrylcholinesterase include neurodegenerative diseases including, but not limited to, Alzheimer's disease and Lou Gherig's disease.

One of ordinary skill in the art also will recognize that the presently disclosed butyrylcholinesterase inhibitors may be generally useful for performing various chemical, biochemical, pharmacological and cellular studies. For example, it may be useful to knock out the activity of butyrylcholinesterase by using the presently disclosed inhibitors and observing the effects. The presently disclosed compounds may be useful in stem cell research.

The present disclosure also provides for methods of treating neurodegenerative diseases with a therapeutically effective amount of a butyrylcholinesterase inhibitor.

Thus, the compounds of the present disclosure may be formulated for oral, buccal, transdermal (e.g., patch), intranasal, parenteral (e.g., intravenous, intramuscular or subcutaneous), ophthalmic or rectal administration or in a form suitable for administration by inhalation or insufflation.

For oral administration, the compounds may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium phosphate); lubricants (e.g., magnesium stearate, talc or silica); disintegrants (e.g., potato starch or sodium starch glycollate); or wetting agents (e.g., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., almond oil, oily esters or ethyl alcohol); and preservatives (e.g., methyl or propyl p-hydroxybenzoates or sorbic acid).

For buccal administration, the compounds may take the form of tablets or lozenges formulated in conventional manner.

The compounds of the present disclosure may be formulated for parenteral administration by injection, including using conventional catheterization techniques or infusion. The compounds may also be formulated for topical ophthalmic administration.

Formulations for injection or topical ophthalmic administration may be presented in unit dosage form, for example in ampules, or in multi-dose containers, optionally with an added preservative. The compounds may take such forms as suspensions, solutions or emulsions in oily or aqueous vehicles, and may contain formulating agents such as suspending, stabilizing and/or dispersing agents. Alternatively, the active ingredient may be in powder form for reconstitution with a suitable vehicle, e.g., sterile pyrogen-free water, before use.

The compounds of the present disclosure may also be formulated in rectal compositions such as suppositories or retention enemas, e.g., containing conventional suppository bases such as cocoa butter or other glycerides.

For intranasal administration or administration by inhalation, the compounds of the present disclosure are conveniently delivered in the form of a solution or suspension from a pump spray container that is squeezed or pumped by the patient. The compounds of the disclosure can also be delivered in the form of an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the disclosure and a suitable powder base such as lactose or starch.

As used herein, the term "effective amount" means an amount of a compound of the present disclosure that is capable of inhibiting the symptoms of a pathological condition described herein by modulation of butyrylcholinesterase activity. The specific dose of a compound administered according to the present disclosure will be determined by the particular circumstances such as the compound administered, the route of administration, the state of being of the patient, and the severity of the pathological condition. A proposed dose of a compound of the present disclosure for oral, parenteral, buccal or topical ophthalmic administration to the average adult human for the treatment of the conditions referred to above is about 0.01 to 50 mg/kg of the active ingredient per unit dose which could be administered, for example, 1 to 4 times per day.

Aerosol formulations for treatment of the conditions referred to above in the average adult human are preferably arranged so that each metered dose or "puff" of aerosol contains 20 µg to 1000 µg of the compound of the disclosure. The overall daily dose with an aerosol will be within the range a 100 µg to 10 mg. Administration may be several times daily, for example 2, 3, 4 or 8 times, giving for example, 1, 2 or 3 doses each time.

### EXAMPLES

### Example 1: Synthesis

### Method A

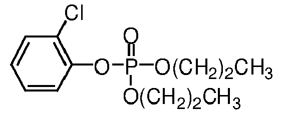

### Representative Procedure for Method A.

To a dry 50 mL round bottom flask were added 10 mL of CH₂Cl₂ and 1.3 mL (2.0 g, 8.2 mmol) of 2-chlorophenyl dichlorophosphate. The solution was cooled to 0 ºC using an ice water bath. Then 2.5 equivalents of alcohol and 2.5 equivalents of pyridine in 10 mL of CH₂Cl₂ were added to the stirring solution via cannulation. The reaction was left to stir overnight at room temperature. The reaction mixture was diluted with 80 mL of diethyl ether and washed three times with 40 mL of 10% HCl. The aqueous layers were combined and washed with 40 mL of CH₂Cl₂. The combined organic layer was washed once with 40 mL of saturated sodium bicarbonate, dried over magnesium sulfate, filtered, concentrated in vacuo, and purified by evaporative distillation.

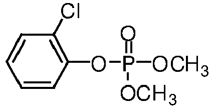

**2-Chlorophenyl dimethyl phosphate.** Following the representative procedure described above and using methanol, 2-chlorophenyl dimethyl phosphate was obtained as a clear oil: 80% yield; b.p.183 ºC/0.2 mm Hg; ¹H NMR (400 MHz, CDCl₃) *δ* 3.92 (d, 6H, 11.4 Hz, CH₃), 7.11-7.15 (m, 1H, Ar-H), 7.52 (td, 1H, 8.2, 1.7 Hz, Ar-H), 7.41-7.42 (m, 1H, Ar-H), 7.43-7.44 (m, 1H, Ar-H); ¹³C NMR (100 MHz, CDCl₃) *δ* 55.22, 55.28, 121.32, 121.34, 125.30, 125.38, 126.03, 128.02, 128.03, 130.65, 146.59, 146.65.

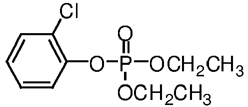

**2-Chlorophenyl diethyl phosphate.** Following the representative procedure described above and using ethanol, 2-chlorophenyl diethyl phosphate was obtained as a clear oil: 100% yield; b.p.180 ºC/ 0.3 mm Hg; ¹H NMR (400 MHz, CDCl₃) *δ* 1.37 (td, 6H, 7.1, 1.1 Hz, CH₃), 4.23-4.32 (m, 4H, CH₂), 7.09-7.13 (m, 1H, Ar-H), 7.24 (td, 1H, 8.2, 1.7 Hz, Ar-H), 7.41 (dt, 1H, 7.9, 1.4 Hz, Ar-H), 7.45 (dt, 1H, 8.2, 1.3 Hz, Ar-H); ¹³C NMR (100 MHz, CDCl₃) *δ* 16.00, 16.03, 16.07, 16.10, 64.97, 65.03, 121.32, 121.35, 125.32, 125.39, 125.76, 127.88, 127.89, 130.57, 146.77, 146.83.

**2-Chlorophenyl di-*n*-propyl phosphate.** Following the representative procedure described above and using *n*-propanol, 2-chlorophenyl di-*n*-propyl phosphate was obtained as a clear oil: 82% yield; b.p. 245 ºC/0.3 mm Hg; ¹H NMR (400 MHz, CDCl₃) *δ* 0.96 (t, 6H, 7.4 Hz, CH₃), 1.74 (sextet, 4H, 7.3 Hz, CH₂C*H*₂ CH₃), 4.11-4.21 (m, 4H, CH₂C*H*₂ CH₃), 7.08-7,13 (m, 1H, Ar-H), 7.24 (td, 1H, 8.2, 1.7 Hz, Ar-H), 7.41 (dt, 1H, 7.9, 1.3 Hz, Ar-H), 7.46 (dt, 1H, 8.2, 1.3 Hz, Ar-H); ¹³C NMR (100 MHz, CDCl₃) *δ* 9.94, 23.56, 23.62, 70.37, 70.44, 121.33, 121.35, 125.34, 125.41, 125.71, 125.73, 127.87, 127.88, 130.55, 146.82, 146.89.

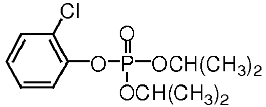

**2-Chlorophenyl di-*i*-propyl phosphate.** Following the general procedure described above and using *i*-propanol, 2-chlorophenyl di-*i*-propyl phosphate was obtained as a clear oil: 70% yield; b.p. 205 ºC/0.7 mm Hg; ¹H NMR (400 MHz, CDCl₃) δ 1.33 (d, 6H, 6.2 Hz, CH₃), 1.38 (d, 6H, 6.2 Hz, CH₃), 4.81 (sept of doublets, 2H, 6.3, 0.8 Hz, C*H*(CH₃)₂), 7.09 (tm, 1H, 8.0 Hz, Ar-H), 7.23 (td, 1H, 8.2, 1.6 Hz, Ar-H), 7.40 (dt, 1H, 7.9, 1.5 Hz, Ar-H), 7.49 (dt, 1H, 8.2, 1.4 Hz, Ar-H); ¹³C NMR (100 MHz, CDCl₃) δ 23.45, 23.47, 23, 50, 23.52, 23.62, 23.64, 23.67, 23.69, 73.94, 74.00, 121.19, 121.21, 125.27, 125.35, 125.43, 127.75, 127.76, 130.48, 147.01, 147.07.

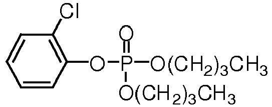

**Di**-***n***-**butyl 2-chlorophenyl phosphate.** Following the general procedure described above and using *n*-butanol, 2-chlorophenyl di-*n*-butyl phosphate was obtained as a clear oil: 52% yield; b.p. 205 ºC/0.6 mm Hg; ¹H NMR (400 MHz, CDCl₃) δ 0.92 (t, 6H, 7.4 Hz, CH₃), δ 1.36-1.45 (m, 4H, 6.2, CH₂CH₂C*H*₂ CH₃), 1,65-1.72 (m, 4H, 6.3, CH₂C*H*₂CH₂ CH₃), 4.15-4.25 (m, 4H, C*H*₂CH₂CH₂ CH₃), 7.09-7.13 (m, 1H, Ar-H), 7.24 (td, 1H, 8.2, 1.6 Hz, Ar-H), 7.41 (dt, 1H, 8.0, 1.4 Hz, Ar-H), 7.45 (dt, 1H, 8.2, 1.3 Hz, Ar-H); ¹³C NMR (100 MHz, CDCl₃) δ 13.53, 18.59, 32.13, 32.20, 68.62, 68.69, 121.32, 121.34, 125.33, 125.41, 125.68, 127.83, 127.84, 130.54, 146.82, 146.88.

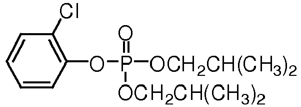

**Di-*i*-butyl 2-Chlorophenyl phosphate.** Following the general procedure described above and using *i*-butanol, di-*i*-butyl 2-chlorophenyl phosphate was obtained as clear oil: 40% yield; b.p. 179 ºC/0.7 mm Hg; ¹H NMR (400 MHz, CDCl₃) δ 0.95 (appt dd, 12H, 6.7, 1.1 Hz, CH₃), 1.99 (septet, 2H, 6.6 Hz, CH), 3.92-4.01 (m, 4H, CH₂), 7.08-7.13 (m, 1H, Ar-H), 7.24 (td, 1H, 8.1, 1.7 Hz, Ar-H), 7.41 (dt, 1H, 8.0, 1.4 Hz, Ar-H), 7.46 (dt, 1H, 8.2, 1.2 Hz, Ar-H); ¹³C NMR (100 MHz, CDCl₃) δ 18.57, 18.59, 18.60, 29.02, 29.09, 74.67, 74.73, 121.37, 121.40, 125.37, 125.44, 125.71, 127.87, 127.88, 130.56, 146.86, 146.91.

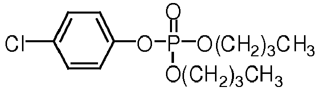

**Di**-***n***-**butyl 4-chlorophenyl phosphate.** Following the general procedure described above using dichloro 4-chlorophenylphosphate and *n*-butanol, di-*n*-butyl 4-chlorophenyl phosphate was obtained as a clear oil: 84%; b.p. 200 ºC/0.2 mm Hg; ¹H NMR (400 MHz, CDCl₃) δ 0.92 (t, 6H, 7.3 Hz, CH₃), 1.35-1.4 (m, 4H, CH₂CH₂C*H*₂ CH₃), 1.67 (quintet, 4H, 6.7 Hz, CH₂C*H*₂CH₂ CH₃), 4.09-4.20 (m, 4H, C*H*₂CH₂CH₂ CH₃), 7.15-7.18 (m, 2H, Ar-H), 7.28-7.31 (m, 1H, Ar-H); ¹³C NMR (100 MHz, CDCl₃) δ 13.41, 18.51, 32.06, 32.13, 68.31, 68.36, 121.24, 129.57, 130.15, 130.16, 149.23, 149.31.

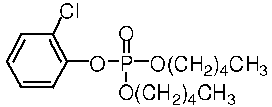

**2-Chlorophenyl di-*n*-pentyl phosphate.** Following the general procedure described above and using *n*-pentanol, 2-chlorophenyl di-*n*-pentyl phosphate was obtained as a clear oil: 88 %; b.p. 185 ºC/0.3 mm Hg; ¹H NMR (400 MHz, CDCl₃) δ 0.89 (t, 6H, 7.2 Hz, CH₃), 1.27-1.39 (m, 8H, CH₂C*H*₂ CH₃), 1.67-1.74 (m, 4H, C*H*₂CH₂CH₂CH₃), 4.14-4.24 (m, 4H, CH₂CH₂CH₂CH₂CH₃), 7.08-7.13 (m, 1H, Ar-H), 7.24 (td, 1H, 8.2, 1.6 Hz, Ar-H), 7.41 (dt, 1H, 7.9, 1.4 Hz, Ar-H), 7.46 (dt, 1H, 8.2, 1.3 Hz, Ar-H); ¹³C NMR (100 MHz, CDCl₃) δ 13.93, 22.17, 27.48, 29.84, 29.90, 68.93, 69.00, 121.33, 121.35, 125.34, 125.41, 125.68, 125.70, 127.85, 127.87, 130.54, 146.81, 146.87.

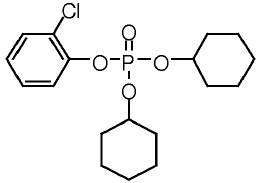

**2-chlorophenyl dicyclohexyl phosphate.** Following the general procedure described above and using cyclohexanol, the crude product was purified by gravity column chromatography (silica gel 1:1, hexane:EtOAc), a second gravity column chromatography (silica gel 7:3, hexane:EtOAc) and evaporative distillation (b.p. 260 ºC/0.10 mm Hg) to afford 2-chlorophenyl dicyclohexyl phosphate as a colorless oil: 70 % yield; ¹H NMR (400 MHz, CDCl₃) *δ* 1.21-1.98 (m, 20H, (OCHC₅*H*₁₀)₂), 4.50-4.59 (m, 2H, (OC*H*)₂), 7.06-7.11 (m, 1H, Ar-*H*), 7.20-7.25 (m, 1H, Ar-*H*), 7.38-7.41 (m, 1H, Ar-*H*), 7.47-7.50 (m, 1H, Ar-*H*); ¹³C NMR (100 MHz, CDCl₃) *δ* 23.32, 25.01, 32.97, 33.02, 33.15, 33.19, 78.40, 78.47, 121.16, 121.18, 125.22, 125.31, 127.65, 127.66, 130.37, 147.01, 147.07.

### Method B

### Representative Procedure for Method B.

A solution of dibutyl phosphite (0.73 g, 3.8 mmol) in 3 mL CCl₄ was added dropwise to a stirring solution containing the substituted phenol (3.0 mmol), 5mL CCl₄, tetra-*n*-butylammonium bromide (0.095 g, 0.3 mmol), NaOH (0.18g, 4.4 mmol) and 5 mL water; it was stirred for an indicated amount of time at room temperature. The reaction mixture was dissolved in 10 mL of CCl₄, extracted with ice-cold distilled water (3 x 10 mL), and each of the aqueous layer was washed with 10mL CCl₄. The combined organic layer was dried under MgSO₄, concentrated in vacuo, and purified by evaporative distillation, flash or gravity chromatography.

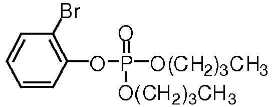

**2-Bromophenyl di-*n*-butyl phosphate.** Following the representative procedure described above using 2-bromophenol (2 hours reaction time), the crude product was purified by evaporative distillation (b.p. 175°C/0.45 mmHg) followed by flash chromatography (silica gel, hexane:EtOAc, 3:2) to give 2-bromophenyl di*n*-butyl phosphate as a pale yellow oil: 56% yield; *R_{f}* = 0.49 (hexane:EtOAc, 1:1); ¹H NMR (400 MHz, CDCl₃) *δ* 0.92 (t, *J* = 7.4 Hz, 6H, CH₂CH₂CH₂C*H*₃), 1.36-1.46 (m, 4H, CH₂CH₂C*H*₂CH₃), 1.65-1.73 (m, 4H, CH₂C*H*₂CH₂CH₃), 4.17-4.26 (m, 4H, C*H*₂CH₂CH₂CH₃), 7.02-7.06 (m, 1H, Ar-H), 7.27-7.31, 1H, Ar-H), 7.47 (dt, *J*=8.2 Hz, 1.3 Hz, 1H, Ar-H), 7.58 (dt, *J* = 8.0 Hz, 1.4 Hz, 1H, Ar-H);¹³C NMR (100MHz, CDCl₃) *δ* 13.46, 18.54, 32.07, 32.14, 68.61, 68.68, 114.29, 114.38, 120.99, 121.01, 125.93, 128.55, 128.56, 133.54, 147.87, 147.93.

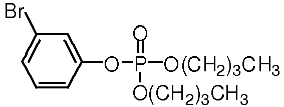

**3-Bromophenyl *n*-dibutyl phosphate.** Following the representative procedure described above using 3-bromophenol, the crude product was purified by gravity chromatography (silica gel, hexane:EtOAc, 4:1) to give 3-bromophenyl di-*n*-butyl phosphate as a clear oil: 38% yield; *R_{f}* = 0.5 (hexane:EtOAc, 1:1); ¹H NMR (400MHz, CDCl₃) *δ* 0.93 (t, J = 7.4 Hz, 6H, OCH₂CH₂CH₂C*H*₃), 1.36-1.45 (m, 4H, OCH₂CH₂C*H*₂CH₃), 1.64-1.71 (m, 4H, OCH₂C*H*₂CH₂CH₃), 4.10-4.20 (m, 4H, OC*H*₂CH₂CH₂CH₃), 7.16-7.23 (m, 2H, Ar-*H*), 7.30-7.33 (m, 1H, Ar-*H*), 7.39-7.40 (m, 1H, Ar-*H*); ¹³C NMR (400 MHz, CDCl₃) *δ* 13.49, 13.50, 18.58, 32.12, 32.19, 68.45, 68.51, 118.75, 118.80, 122.56, 123.47, 123.52, 128.16, 130.70, 151.21, 151.28.

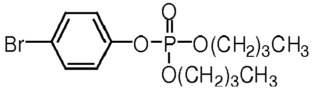

**4-Bromophenyl di-*n*-butyl phosphate.** Following the representative procedure described above using 4-bromophenol, the crude product was purified by gravity chromatography (silica gel, 4:1, hexane:EtOAc) to give 4-bromophenyl di-*n*-butyl phosphate as a clear oil: 32% yield; *R_{f}* = 0.06 (hexane:EtOAc, 4:1); ¹H NMR (400MHz, CDCl₃) *δ* 0.93 (t, J = 7.4 Hz, 6H, OCH₂CH₂CH₂C*H*₃), 1.35-1.44 (m, 4H, OCH₂CH₂C*H*₂CH₃), 1.63-1.71 (m, 4H, OCH₂C*H*₂CH₂CH₃), 4.08-4.19 (m, 4H, OC*H*₂CH₂CH₂CH₃), 7.09-7.13 (m, 2H, Ar-*H*), 7.43-7.47 (m, 2H, Ar-*H*); ¹³C NMR (400 MHz, CDCl₃) *δ* 13.49, 13.50, 18.57, 32.13, 32.19, 68.40, 68.46, 117.81, 117.83, 121.74, 121.79, 132.65, 149.86, 149.93.

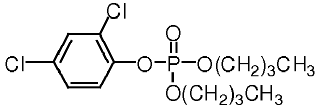

**Di**-***n***-**butyl 2,4-dichlorophenyl phosphate.** Following the representative procedure described above, the crude product was purified by gravity chromatography (silica gel, hexane EtOAc, 9:1) to give di-*n*-butyl 2,4-dichlorophenyl phosphate as a light yellow oil: 22.26% yield; *R_{f}* = 0.12 (silica gel, hexane: EtOAc, 9:1); ¹H NMR (400MHz, CDCl₃) *δ* 0.930 (t, J=7.4 Hz, 6H, OCH₂CH₂H₂C*H*₃)₂), 1.36-1.46 (m, 4H, (OCH₂CH₂C*H*₂CH₃)₂), 1.65-1.72 (m, 4H, (OCH₂C*H*₂CH₂CH₃)₂), 4.14-4.24 (m, 4H, (OC*H*₂CH₂CH₂CH₃)₂), 7.26 (dd, 1H, J=8.9, 2.5 Hz, Ph-*H*), 7.40 (dd, 1H, J=8.8, 1.1 Hz, Ph-*H*), 7.42 (dd, J=2.6, 1H, 1.1 Hz, Ph-*H*) ; ¹³C NMR (100MHz, CDCl₃) *δ* 13.47, 18.54, 32.08, 32.14, 68.73, 68.80, 122.08, 122.10, 126.21, 126.29, 127.92, 130.20, 130.40, 130.42, 145.60, 145.66.

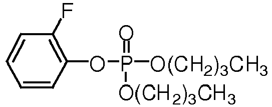

**Di**-***n***-**butyl 2-fluorophenyl phosphate.** Following the representative procedure described above using 2-fluorophenol, the crude product was purified by gravity chromatography (silica gel, hexane:EtOAc, 4:1) to give di-*n*-butyl 2-fluorophenyl phosphate as a clear oil: 48.25% yield; *R_{f}* = 0.25 (silica gel, hexane:EtOAc, 4:1); ¹H NMR (400MHz, CDCl₃) *δ* 0.93 (t, J=7.4 Hz, 6H, (OCH₂CH₂CH₂C*H*₃)₂), 1.36-1.46 (m, 4H, (OCH₂CH₂C*H*₂CH₃)₂), 1.65-1.72 (m, 4H, (OCH₂C*H*₂CH₂CH₃)₂), 4.13-4.24 (m, 4H, (OC*H*₂CH₂CH₂CH₃)₂), 7.07-7.17 (m, 3H, Ph-*H*), 7.35-7.40 (m, 1H, Ph-*H*); ¹³C NMR (100MHz, CDCl₃) *δ* 13.44, 13.48, 18.52, 32.08, 32.15, 68.47, 68.54, 116.72, 116.91, 122.31, 122.34, 124.40, 124.41, 124.44, 124.45, 125.74, 125.75, 125.80 , 125.82, 138.36, 138.42,138.48, 138.55, 152.24, 152.30, 154.71, 154.77.

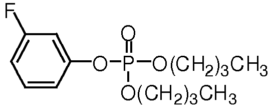

**Di**-***n***-**butyl 3-fluorophenyl phosphate.** Following the representative procedure described above using 3-fluorophenol, the crude product was purified by gravity chromatography (silica gel, hexane:EtOAc, 4:1) to give di-*n*-butyl 3-fluorophenyl phosphate as a clear oil: 31% yield; *R_{f}* = 0.13 (silica gel, hexane:EtOAc, 4:1); ¹H NMR (400 MHz, CDCl₃) *δ* 0.93 (t, J= 7.4 Hz, 6H, OCH₂CH₂CH₂C*H*₃), 1.36-1.45 (m, 4H, OCH₂CH₂C*H*₂CH₃), 1.64-1.71 (m, 4H, OCH₂C*H*₂CH₂CH₃), 4.10-4.21 (m, 4H, OC*H*₂CH₂CH₂CH₃), 6.87-6.92 (m, 1H, Ar-*H*), 6.97 (dtd, 1H, J=9.7, 2.3, 0.8 Hz, Ar-*H*), 7.02-7.04 (m, 1H, Ar-*H*), 7.29 (td, 1H, 8.2, 6.6 Hz, Ar-*H*); ¹³C NMR (400 MHz, CDCl₃) *δ* 13.48, 18.57, 32.12, 32.19, 68.41, 68.48, 107.94, 107.99, 108.19, 108.24, 111.86, 112.07, 115.68, 115.71, 115.73, 115.76, 130.36, 130.44, 151.50, 151.57, 151.67, 161.76, 164.21.

**Di**-***n***-**butyl 3-nitrophenyl phosphate.** Following the representative procedure 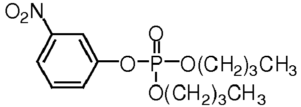 described above, the crude product was purified by gravity chromatography (silica gel, hexane:EtOAc, 4:1) to give di-*n-*butyl 3-nitrophenyl phosphate as a light yellow oil: 53% yield; *R_{f}* = 0.18 (silica gel, hexane:EtOAc, 4:1); ¹H NMR (400MHz, CDCl₃) *δ* 0.94 (t, J=7.3 Hz, 6H, (OCH₂CH₂CH₂C*H*₃)₂), 1.37-1.46 (m, 4H, (OCH₂CH₂C*H*₂CH₃)₂), 1.67-1.7 (m, 4H, (OCH₂C*H*₂CH₂CH₃)₂), 4.14-4.24 (m, 4H, (OC*H*₂CH₂CH₂CH₃)₂), 7.51-7.56 (m, 1H, Ar-H), 7.61 (ddt, 1H, J=8.2, 2.2, 1.1 Hz, Ar-H), 8.05-8.08 (m, 2H, Ph-*H*); ¹³C NMR (100MHz, CDCl₃) *δ* 13.37, 13.41, 18.49, 32.03, 32.10, 68.67, 68.73, 115.48, 115.53, 119.79, 126.26, 126.31, 130.30, 148.85, 151.10, 151.17.

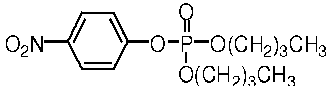

**Di**-***n***-**butyl 4-nitrophenyl phosphate.** Following the representative procedure described above using 4-nitrophenol (1 hour reaction time), the crude product was purified by gravity chromatography (silica gel, hexane:EtOAc, 3:2) to give di-*n-*butyl 4-nitrophenyl phosphate as a pale yellow oil: 41% yield; *R_{f}* = 0.31 (hexane:EtOAc, 3:2); ¹H NMR (400 MHz, CDCl₃) *δ* 0.93 (t, *J* = 7.4 Hz, 6H, CH₂CH₂CH₂C*H*₃), 1.36-1.45 (m, 4H, CH₂CH₂C*H*₂CH₃), 1.66-1.73 (m, 4H, CH₂C*H*₂CH₂CH₃), 4.13-4.23 (m, 4H, C*H*₂CH₂CH₂CH₃), 7.36-7.40 (m, 2H, Ar-H), 8.23-8.27 (m, 2H, Ar-H);¹³C NMR (100MHz, CDCl₃) *δ* 13.46, 18.56, 32.09, 32.16, 68.79, 68.85, 120.47, 120.51, 125.65, 144.60, 155.58, 155.65. 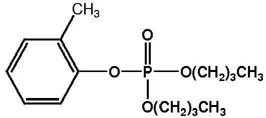 **Di**-***n***-**butyl 2-methylphenyl phosphate.** Following the representative procedure above using 2-methylphenol, the crude product was purified by gravity chromatography (silica gel, 4:1 hexane:EtOAc) to give di-*n*-butyl 2-methylphenyl phosphate as light yellow oil: 46% yield; *R_{f}* (silica gel, 80:20 hexane:EtOAc) = 0.18; ¹H NMR (400 MHz, DCCl₃) *δ* 0.92 (t, J= 7.4Hz, 6H, (OCH₂CH₂CH₂C*H*₃)₂), 1.35-1.45 (m, 4H, (OCH₂CH₂C*H*₂CH₃)₂), 1.64-1.71 (m, 4H, (OCH₂C*H*₂CH₂CH₃)₂), 2.31 (s, 3H, Ar-C*H*₃), 4.09-4.20 (m, 4H, (OC*H*₂CH₂CH₂CH₃)₂), 7.04-7.08 (m, 1H, Ar-*H*), 7.14 (dd, J=7.6, 2.0 Hz, 1H, Ar-H), 7.18-7.20 (m, 1H, Ar-*H*), 7.27-7.29 (m, 1H, Ar-*H*); ¹³C NMR (100MHz, CDCl₃) δ 13.48, 16.30, 16.31, 18.58, 32.16, 32.24, 68.14, 68.205, 119.64, 119.66, 124.80, 124.82, 126.92, 126.94, 129.14, 129.21, 131.22, 149.21, 149.28. MS m/z 300; Calc. 300.

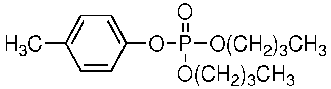

**Di**-***n***-**butyl 4-methylphenyl phosphate.** Following the general procedure above using 4-mwthylphenol, the crude product was purified by gravity chromatography (silica gel, 4:1 hexane:EtOAc) to give di-*n*-butyl 4-methylphenyl phosphate as a clear oil :38% yield; *R_{f}* (silica gel, 80:20 hexane:EtOAc) = 0.20; ¹H NMR (400 MHz, DCCl₃) 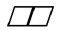 0.92 (t, J=7.4 Hz, 6H, (OCH₂CH₂CH₂C*H*₃)₂), 1.35-1.44 (m, 4H, (OCH₂CH₂C*H*₂CH₃)₂), 1.63-1.70 (m, 4H, (OCH₂C*H*₂CH₂CH₃)₂), 2.32 (s, 3H, Ar-C*H*₃), 4.08-4.19 (m, 4H, (OC*H*₂CH₂CH₂CH₃)₂), 7.08-7.13 (m, 4H, Ar-*H*); ¹³C NMR (100MHz, CDCl₃) *δ* 13.48, 18.57, 20.65, 20.67, 32.14, 32.21, 68.09, 68.15, 119.61, 119.66, 130.04, 134.41, 134.42, 148.54, 148.61. MS m/z 300; Calc. 300.

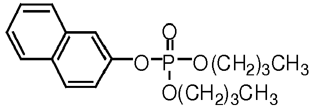

**Di**-***n***-**butyl 2-naphthyl phosphate.** Following the representative procedure described above using 2-naphthol, the crude product was purified by evaporative distillation (b.p. 235 ºC/0.23 mm Hg) followed by flash column chromatography (silica gel, hexane:EtOAc, 4:1) to give di-*n*-butyl 2-naphthyl phosphate as a yellow oil: 54.5% yield; Rf = 0.30 (hexane:EtOAc, 4:1); ¹H NMR (400 MHz, CDCl₃) *δ* ¹H NMR (400 MHz, CDCl3) δ 0.91 (t, *J* = 7.4 Hz, 6H (OCH₂CH₂CH₂C*H*₃)₂), 1.36-1.45 (m, 4H (OCH₂CH₂C*H*₂CH₃)₂, 1.65-1.72 (m, 4H (OCH₂C*H*₂CH₂CH₃)₂), 4.12-4.23 (m, 4H (OC*H*₂CH₂CH₂CH₃)₂), 7.36 (dd, *J* = 8.9, 2.4 Hz, 1H, Ar-*H*), 7.41-7.50 (m, 2H, Ar-*H*), 7.69 (s, 1H, Ar-*H*), 7.80 (t, J = 9.6 Hz, 3H, Ar-*H*).

### Additional Methods

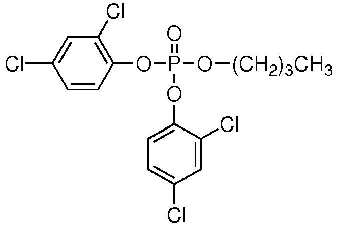

***n***-**Butyl bis(2,4-dichlorophenyl) phosphate.** To a round bottom flask charged with pyridine (0.06 mL, 0.00073 mol) in 2 mL dry CH₂Cl₂ was added n-butyl alcohol (0.067 mL, 0.00073 mol). This solution was added drop-wise via cannulation at 0°C to a solution of bis(2,4dichlorophenyl) phosphorochloridate (0.15g, 0.00036 mol) in 3 mL dry CH₂Cl₂. The reaction was allowed to react for 24 hrs at room temperature. The reaction was quenched by extracting with 2 mL 10 % HCl, 5 mL saturated NaHCO₃ and 10 mL ethyl ether. The aqueous phase was extracted 3X5 mL with diethyl ether. The combined organic layer was dried over MgSO₄, filtered and concentrated in vacuo. The crude product was purified by gravity column chromatography (silica gel, EtOAc) to give n-butyl bis(2,4-dichlorophenyl) phosphate as an oil: 10% yield; *R_{f}* = 0.36 (EtOAc); ¹H NMR (400 MHz, CDCl₃) *δ* 0.93 (t, 3H, J= 7.6Hz, CH₃), 1.36-1.46 (m, 2H, CH₂CH₂C*H*₂CH₃), 1.69-1.76 (m, 2H, CH₂C*H*₂CH₂CH₃), 4.37 (q, 2H, J= 6.8 Hz, C*H*₂CH₂CH₂CH₃), 7.23 (dd, J= 8.8, 2.4 Hz, 1H, Ar-H), 7.40 (dd, 1H, J= 8.8, 1.2 Hz, Ar-H), 7.44 (dd, 1H, J= 2.8, 1.2 Hz, Ar-H); ¹³C NMR (100 MHz, CDCl₃) *δ* 13.44, 18.49, 31.98, 32.05, 70.35, 70.43, 122.23,122.26, 126.41, 126.48, 128.04, 128.05, 130.42, 131.18, 131.20, 145.14, 145.20.

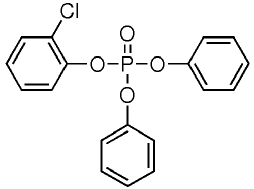

**2-Chlorophenyl diphenyl phosphate.** To a round bottom flask charged with NaH (0.1662g of a 60% dispersion in mineral oil, approximately 0.42 mmol) in 3 mL dry THF was added phenol (0.383g, 0.40 mmol) in 2 mL dry THF. Then this solution was added drop wise via cannulation to a solution of 2-chlorophenyl dichlorophosphate (0.5g, 0.20 mmol) in 5mL dry THF at room temperature. The mixture was allowed to react overnight and quenched by adding 10 ml of saturated NaHCO₃ and 10 mL of diethyl ether. The aqueous layer was extracted with diethyl ether (3X 10mL). The combined organic layer was dried over MgSO₄, filtered and concentrated in vacuo. The crude product was purified by gravity column chromatography (silica gel, hexane:EtOAc, 7:3) to give 2-chlorophenyl diphenyl phosphate as an orange oil: 10% yield; *R_{f}* = 0.22 (silica, hexane:EtOAc, 7:3); ¹H NMR (400 MHz, CDCl₃) *δ* 7.14 (t,1H, J= 8 Hz, Ar-H), 7.22 (7, 3H, J= 7.2 Hz, Ar-H), 7.28 (d, 4H, J= 8Hz, Ar-H), 7.36 (t, 4H, J= 8 Hz, Ar-H), 7.41-7.44 (m, 2H, Ar-H); ¹³C NMR (100 MHz, CDCl₃) *δ* 120.18, 120.23, 121.36, 121.39, 125.50, 125.58, 125.70, 125.71, 126.31, 127.92, 127.93, 129.83, 130.77, 146.53, 146.59, 150.29, 150.37.

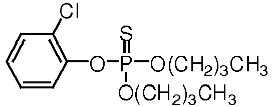

**Dibutyl 2-chlorophenyl thiophosphate.** A mixture containing *n*-butanol (0.5 mL, 5.4 mmol) and distilled pyridine (0.45 mL, 5.7 mmol) in 5 mL CH₂Cl₂ was added dropwise to 2-chlorophenyl dichlorothiophosphate (0.56 g, 2.1 mmol) dissolved in 15 mL dry CH₂Cl₂, The reaction mixture was allowed to stir for 4 days, followed by dilution with 15 mL CH₂Cl₂, and extraction with 10% HCl (1 x 20 mL) and saturated NaHCO₃ (3 x 15 mL). The organic layer was dried under MgSO₄, concentrated in vacuo, and purified by evaporative distillation to yield a pale yellow oil; 73% yield: b.p. 145°C/0.1 mmHg; ¹H NMR (400 MHz, CDCl₃) *δ* 0.94 (t, *J* = 7.4 Hz, 6H, CH₂CH₂CH₂C*H*₃), 1.39-1.48 (m, 4H, OCH₂CH₂C*H*₂CH₃), 1.68-1.75 (m, 4H, CH₂C*H*₂CH₂CH₃), 4.22 (dt, *J* = 8.8 Hz, 6.5 Hz, 4H, C*H*₂CH₂CH₂CH₃), 7.10-7.14 (m, 1H, Ar-H), 7.22-7.26 (m, 1H, Ar-H), 7.37 (dt, *J* = 8.2 Hz, 1.5 Hz, 1H, Ar-H), 7.42 (dt, *J* = 8.0 Hz, 1.3 Hz, 1H, Ar-H);¹³C NMR (100MHz, CDCl₃) *δ* 13.55, 18.70, 31.95, 32.03, 68.93, 69.00, 122.19, 122.22, 125.82, 125.84, 126.11, 126.18, 127.53, 127.54, 130.51, 146.97, 147.04.

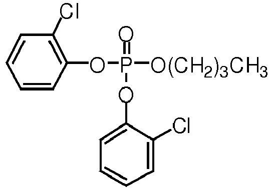

**Butyl bis(2-chlorophenyl) phosphate.** A mixture containing *n*-butanol (0.17 mL, 1.86 mmol), and distilled pyridine (0.15 mL, 1.86 mmol) in 5 mL CH₂Cl₂ was added dropwise to bis(2-chlorophenyl) chlorophosphate (0.63 g, 1.56 mmol) dissolved in 15 mL dry CH₂Cl₂, The reaction mixture was allowed to stir for 24 hours, followed by dilution with 15 mL CH₂Cl₂, extraction with 10% HCl (1 x 20 mL) and saturated NaHCO₃ (3 x 15 mL), and was dried under MgSO₄. Upon concentration in vacuo, a pale yellowish oil was obtained in 77% yield: ¹H NMR (400 MHz, CDCl₃) *δ* 0.92 (t, *J* = 7.4 Hz, 3H, CH₂CH₂CH₂C*H*₃), 1.37-1.46 (m, 2H, CH₂CH₂C*H*₂CH₃), 1.70-1.77 (m, 2H, CH₂C*H*₂CH₂CH₃), 4.37-4.42 (m, 2H, C*H*₂CH₂CH₂CH₃), 7.11-7.16 (m, 2H, Ar-H), 7.23 (dd, *J* = 8.2 Hz, 1.7 Hz, 2H, Ar-H), 7.42 (dt, *J* = 7.9 Hz, 1.4 Hz, 2H, Ar-H), 7.47 (dt, *J* = 8.2Hz, 1.4 Hz, 2H, Ar-H); ¹³C NMR (100MHz, CDCl₃) *δ* 13.45, 18.49, 32.01, 32.07, 69.97, 70.05, 121.49, 121.51, 125.47, 125.55, 126.14, 126.16, 127.87, 127.88, 130.63, 146.50, 146.56.

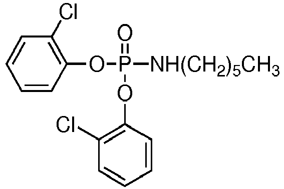

**Bis(2-chlorophenyl) *N*-hexyl phosphoramidate.** Into a flame-dried 50 mL round bottom flask was added 2.85 mmol of bis(2-chlorophenyl) chlorophosphate dissolved in 4 ml of CH₂Cl₂. This mixture was stirred for fifteen minutes at 0 °C. Into a separate flame dried 50 mL round bottom flask was placed 1.6 equivalents of *n*-hexylamine dissolved in 4 ml of CH₂Cl₂ and 1.9 equivalents of pyridine. This solution was allowed to stir for ten minutes at 0 °C. The hexylamine/pyridine solution was then added to bis(2-chlorophenyl) chlorophosphate drop wise over a ten minute period via syringe. The reaction was stirred at 0 °C for ten minutes then stirred at room temperature overnight. Reaction mixture was diluted with16 ml of CH₂Cl₂ and washed three times with 20 ml of 10% HCl. The organic layer was washed two times with 18 ml of saturated NaHCO₃ solution, dried over MgSO₄, and concentrated in vacuo to give bis(2-chlorophenyl) *N-*hexyl phosphoramidate as a golden oil: 71% yield; ¹H NMR (400 MHz, CDCl₃) *δ* 0.85 (t, 3H, J=7.0 Hz, CH₃), 1.19-1.28(m, 6H, C*H*₂C*H*₂C*H*₂CH₃), 1.43-1.50 (m, 2H, NHCH₂C*H*₂), 3.1-3.2 (dq, 2H, J=10.8, 7.0 Hz, NHC*H*₂), 3.56 (dt, 1H, J= 13.4, 6.7 Hz, NH), 7.07-7.11 (m, 2H, Ar-H), 7.21 (td, 2H, J=7.9, J=1.6, Ar-H), 7.40 (dt, 2H, J= 8.0, 1.3 Hz, Ar-H), 7.54 (dt, 2H, J=8.2, 1.3 Hz, Ar-H); ¹³C NMR (100 MHz, CDCl₃) *δ* 13.95, 22.48, 26.06, 31.33, 31.40, 41.87, 121.74, 121.76, 125.45, 125.52, 125.66, 125.67, 127.74, 127.75, 130.46, 146.81, 146.87.

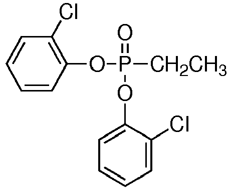

**Bis(2-chlorophenyl) ethylphosphonate.** A dried round bottom flask was charged with 0.60 mL (0.81 g, 5.5 mmol) of ethyl phosphonic dichloride and 9 mL of dry THF. To another dried round bottom flask sodium hydride (0.558 g of a 60% dispersion in mineral oil, approximately 14 mmol), 7 mL of dry THF and 2.1 equivalents (1.20 mL, 11.6 mmol) of 2-chlorophenol were added. The sodium 2-chlorophenoxide was added to the stirring phosphonic dichloride solution via cannulation. The mixture was allowed to react overnight at room temperature. The reaction mixture was dissolved in 50 mL of diethyl ether and washed three times with 10 mL of saturated sodium bicarbonate. The organic layer was then washed three times with 10 mL of saturated sodium chloride solution, dried over magnesium sulfate, filtered, and concentrated in vacuo to give 2.08 g of a pale yellow oil. The oil was purified by flash column chromatography (silica gel, 3:7, EtOAc:hexane) and evaporatively distilled (220°C/0.2 mm Hg) to afford bis(2-chlorophenyl) ethylphosphonate as a clear colorless oil: 28%; GCMS (*m*/*z*) 139 (100%), 295 (M⁺, 85%); *R_{f}*=0.41 (2:3, hexane:EtOAc); ¹H NMR (400 MHz, CDCl₃) *δ* 1.45 (dt,3H, J= 22.0, 7.6 Hz, CH₂C*H*₃), 2.26 (dq, 2H, J = 18.5, 7.7 Hz, C*H₂*CH₃), 7.09-7.14 (m, 1H, Ar-*H*), 7.17-7.22 (1H, Ar-*H*), 7.33 (dt, 1H, J = 8.2 Hz, 1.5 Hz, Ar-*H*), 7.42 (ddd, 1H, J = 8.0, 1.6, 0.8 Hz, Ar-*H*); ¹³C NMR (100 MHz, CDCl₃) *δ* 19.29, 20.70, 122.26, 122.29, 125.67, 125.73, 125.95, 125.96, 127.81, 127.83, 130.57, 146.22, 146.30.

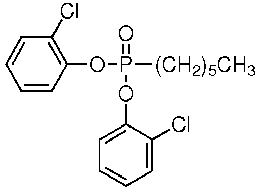

**Bis(2-chlorophenyl) hexylphosphonate.** Following the procedure above for bis(2-chlorophenyl) ethylphosphonate and using hexyl phosphonic dichloride, the crude product was purified by evaporative distillation (160-185 °C/0.25 mm Hg) to remove 2-chlorophenol to afford bis(2-chlorophenyl) hexylphosphonate as a clear colorless oil: 50% yield; ¹H NMR (400 MHz, CDCl₃) *δ* 0.88-0.91 m, 3H, CH₃), 1.30-1.35 (m, 4H, (CH₂)₃(C*H*₂)₂CH₃), 1.44-1.52 (m, 2H, (CH₂)₂C*H*₂(CH₂)₂CH₃), 1.84-1.95 (m, 2H, CH₂C*H*₂ (CH₂)₃CH₃), 2.18-2.27 (m, 2H, C*H*₂ (CH₂)₄CH₃), 7.09-7.13 (m, 1H, Ar-H),7.19 (td, 1H, J=8.0, 1.7 Hz, Ar-H), 7.33 (dt, 1H, J=8.2, 1.5 Hz, Ar-H), 7.40-7.43 (m, 1H, Ar-H).

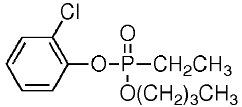

**Butyl 2-chlorophenyl ethylphosphonate.** To a dried round bottom flask 0.420 g (1.27 mmol) of bis(2-chlorophenyl) ethylphosphonate in 10 mL of dry THF with stirring was added. To another dried round bottom flask sodium hydride (0.0645 g of a 60% dispersion in mineral oil, approximately 1.6 mmol) and 3 mL of dry THF were added, followed by 1.1 equivalents (0.125 mL, 1.37 mmol) of anhydrous 1-butanol. The sodium butoxide was added to the stirring phosphonate solution via cannulation. After allowing the mixture to react for 36 hours at room temperature, an additional 0.32 equivalents of sodium butoxide was added to the reaction mixture via syringe. The reaction mixture was allowed to react overnight at room temperature. The reaction mixture was diluted in 50 mL of diethyl ether and washed three times with 10 mL of saturated sodium bicarbonate solution. The organic layer was then washed three times with 10 mL of saturated sodium chloride solution, dried over magnesium sulfate, filtered, and concentrated in vacuo to give 0.09 g of a yellow oil. The crude product was purified by gravity column chromatography (gravity grade silica gel, 3:2, EtOAc:hexane) to afford butyl 2-chlorophenyl ethylphosphonate as a clear colorless oil: 2% yield, GCMS (*m*/*z*) 185 (100%), 276 (M⁺, 1%); ¹H NMR (400 MHz, CDCl₃) *δ* 0.91 (t, 3H, J=7.2 Hz, OCH₂CH₂CH₂C*H*₃), 1.24-1.43 (m, 5H, OCH₂CH₂C*H*₂CH₃ and PCH₂C*H*₃), 1.60-1.69 (m, 2H, OCH₂C*H*₂CH₂CH₃), 1.94-2.04 (m, 2H, PC*H*₂CH₃), 4.06-4.24 (m, 2H, OC*H*₂(CH₂)₂CH₃), 7.10 (t, 1H, 5 Hz, Ar-H),7.21-7.26 (m, 1H, Ar-H), 7.40 (dd, 1H, J=7.9, 1.4Hz, Ar-H), 7.46 (d, 1H, J=12.8 Hz, Ar-H).

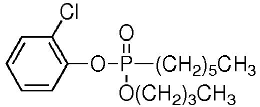

**Butyl 2-chlorophenyl hexylphosphonate.** Following the procedure above for butyl 2-chlorophenyl ethylphosphonate and using bis(2-chlorophenyl) hexylphosphonate, the crude product was purified by evaporative distillation to remove 2-chlorophenol (140-165 °C/0.18 mm Hg) to afford butyl 2-chlorophenyl hexylphosphonate as an oil: 6% yield; ¹H NMR (400 MHz, CDCl₃) *δ* 0.89 (t, 3H, J=6.9 Hz, CH₃), 0.91 (t, 3H, J=7.4 Hz, CH₃), 1.27-1.45 (m, 8H, OCH₂CH₂C*H*₂CH₃, (CH₂)₂(C*H*₂)₃CH₃), 1.60-1.77 (m, 4H, OCH₂C*H*₂CH₂CH₃, CH₂C*H*₂(CH₂)₃CH₃), 1.92-2.00 (m, 2H, C*H*₂(CH₂)₄CH₃), 4.05-4.21 (m, 2H, OC*H*₂CH₂CH₂CH₃), 7.07-7.11 (m, 1H, Ar-H),7.23 (td, 1H, J=8.0, 1.6 Hz, Ar-H), 7.39-7.41 (m, 1H, Ar-H), 7.45 (dt, 1H, J=8.2, 1.4 Hz, Ar-H).

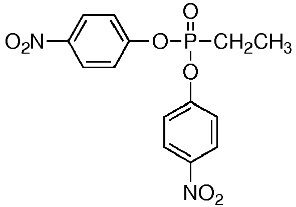

**Bis (p-nitrophenyl) ethylphosphonate.** To a round bottom flask charged with NaH (0.333g, 0.0138mol) in 5mL dry THF was added 4-nitrophenol (1.1359g, 0.0081 mol) in 5mL dry THF. A solution of ethyl phosphonic dichloride (0.6g, 0.0040 mol) in 5mL dry THF was then added via cannulation at room temperature. The reaction was allowed to react overnight. The reaction was quenched by adding 15 mL of CH₂Cl₂ and 15mL of saturated NaHCO₃. The aqueous layer was extracted with methylene chloride 3X20mL. The combined organic layer was dried over MgSO₄, filtered and concentrated in vacuo. The crude solid product was dissolved in CH₂Cl₂ and the organic layer extracted several times with saturated NaHCO₃ The organic layer was dried over MgSO₄, filtered and concentrated in vacuo to give bis(p-nitrophenyl) ethylphosphonate as a white solid (mp 157-159°C): 15% yield; ¹H NMR (400 MHz, CDCl₃) *δ* 1.40 (dt, 3H, J= 22.4, 7.6 Hz. CH₃), 2.22 (dq, CH₂, J= 18.4, 7.6 Hz,CH₂), 7.36-7.39 (m, 2H, Ar-H), 8.23-8.26 (m, 2H, Ar-H); ¹³C NMR (100 MHz, CDCl₃) *δ* 6.30, 6.38, 19.01, 20.42, 120.97, 121.02, 125.83, 144.98, 154.75, 154.84.

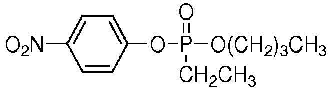

**Butyl p-nitrophenyl ethylphosphonate.** To a round bottom flask with NaH (0.007g of a 60% dispersion in mineral oil, approximately 0.18 mmol) in 1mL dry THF was added dry 1-butanol (0.016mL, 0.18 mmol). This solution was added drop wise via cannulation to a solution of bis(p-nitrophenyl) ethylphosphonate (0.093mg, 0.26 mmol) in 3mL dry THF in an ice/water bath. The mixture was allowed to react at room temperature overnight. The reaction was quenched by adding 5 mL of methylene chloride and 5 mL of NaHCO₃. The aqueous layer was extracted with methylene chloride (3X5mL). The organic layer was combined and dried over MgSO₄, filtered and concentrated in vacuo. The crude product was purified by gravity column chromatography (silica gel, 70:30, EtOAc:hexane) to give butyl p-nitrophenyl ethylphosphonate as an oil: 26% yield; Rf = 0.3 (silica gel, 70:30, EtOAc:hexane); ¹H NMR(400 MHz, CDCl₃) *δ* 0.92 (t, J= 7.4 Hz, 3H, OCH₂CH₂CH₂C*H*₃), 1.27 (dt, J= 21.1, 7.7 Hz, 3H, PCH₂C*H*₃), 1.34-1.42 (m, 2H, OCH₂CH₂C*H*₂CH₃), 1.61-1.68 (m, 2H, OCH₂C*H*₂CH₂CH₃), 1.96 (dq, J= 18.3, 7.6 Hz, 2H, PC*H*₂CH₃), 4.08 (dq, J=10.1, 6.6 Hz, 1H, OC*H*(H)CH₂CH₂CH₃), 4.14-4.22(m, 1H, OCH(*H*)CH₂CH₂CH₃), 7.39(dd, J= 9.3, 1.1 Hz, 2H, Ar-H), 8.24 (d, J= 8.9 Hz, 2H, Ar-H).

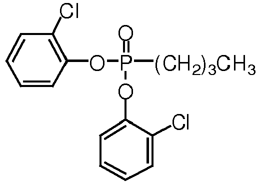

**Bis(2-chlorophenyl) *n***-**butylphosphonate**. Into a dried three-neck 100 mL round bottom flask equipped with an addition funnel and a reflux condenser were added magnesium turnings (4.0 g/17 mmol) followed by 1 ml of dry diethyl ether. To this mixture was added drop wise with stirring at room temperature a solution of 1-bromobutane (1.8 mL/17 mmol) in 4 mL of diethyl ether over 30 minutes. After Grignard reagent formation was complete, 2-chlorophenyl dichlorophosphate (1.23 g/5.0 mmol) in 2 mL diethyl ether was added drop wise at rt. After allowing the mixture to stir for 2.5h, it was worked up by addition of saturated aqueous NH₄Cl and the organic layer separated. The aqueous layer was extracted with diethyl ether (3X) and the combined organic layer extracted with brine, dried over MgSO₄, filtered and concentrated in vacuo. The yellowish oil was purified by flash chromatography (silica gel, 3:2, hexane: EtOAc) to give bis(2-chlorophenyl) butylphosphinate as an oil: 21% yield; *Rf*=0.35 (3:2, hexane:EtOAc); ¹H NMR (400 MHz, CDCl₃) *δ* 0.96 (t, 3H, J=7.4 Hz, CH₂CH₂CH₂C*H*₃), 1.51 (sextet, 2H, J=7.4 Hz, CH₂CH₂C*H*₂CH₃), 1.84-1.95 (m, 2H, CH₂C*H*₂CH₂CH₃), 2.19-2.28 (m, 2H, C*H*₂CH₂CH₂CH₃), 7.08-7.13 (m, 1H, Ar-H), 7.19 (td, 1H, J=8.0, 1.7 Hz, Ar-H), 7.33 (dt, 1H, J=8.2, 1.4 Hz, Ar-H), 7.41 (ddd, 1H, J=7.9, 1.6. 0.8 Hz, Ar-H); ¹³C NMR (100MHz, CDCl₃) *δ* 16.46, 16.64, 17.16, 17.21, 18.55, 19.94115.25, 115.28, 118.63, 118.69, 118.90, 118.92, 120.79, 120.80, 123.54, 139.24, 139.32.

**2-Chlorophenyl di-*n*-butylphosphinate.** The title compound was also isolated 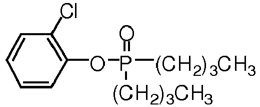 during the chromatographic purification of bis(2-chlorophenyl) *n-*butylphosphonate. Therefore, the 2-chlorophenyl di-*n-*butylphosphinate was further purified by flash chromatography (silica gel, 4:1 to 3:2, hexane: EtOAc) to give the title compound as an oil: 9% yield; *Rf*=0.23 (3:2, hexane:EtOAc); 0.92 (t, 6H, J= 7.2 Hz, CH₂CH₂CH₂C*H*₃), 1.42 (sextet, 4H, J=7.4 Hz, CH₂CH₂C*H*₂CH₃), 1.54-1.75 (m, 4H, CH₂C*H*₂CH₂CH₃), 1.84-1.96 (m, 4H, C*H*₂CH₂CH₂CH₃), 7.06-7.10 (m, 1H, Ar-H), 7.22 (td, 1H, J=7.9, 1.7 Hz, Ar-H), 7.39 (dd, 1H, J=8.0, 1.6 Hz, Ar-H), 7.54 (dt, 1H, J=8.2, 1.4 Hz, Ar-H).

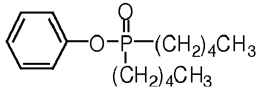

**Phenyl di**-***n***-**pentylphosphinate**. Into a dried three-neck 100 mL round bottom flask was added 1.5 equivalents of magnesium turnings and 4 ml of dry THF. This mixture was stirred at room temperature. To a second dried round bottom flask was added 1.5 equivalents of 1-bromopentane diluted in 4 mL of THF. This mixture was stirred for five minutes and transferred to an addition funnel. The 1-bromopentane was added to the magnesium turnings drop wise over a thirty minute period at room temperature. The Grignard reagent begin to form fifteen minutes after the addition was completed. To a third dried round bottom flask was added phenyl dichlorophosphate dissolved in 1 ml of THF and transferred to the addition funnel. The phenyl dichlorophosphate/THF was added to the 1-bromopentane mixture over a fifteen minute period at 0 °C. The reaction mixture was stirred at 0 °C for 35 minutes and then removed from the ice bath and allowed to continue stirring for 1.5 hours at room temperature. A golden liquid was obtained and was worked up using saturated NH₄Cl. The organic layer was isolated and washed with brine and dried over MgSO₄, filtered, concentrated in vacuo and purified by flash chromatography (silica gel, 3:2, hexane:EtOAc) to give phenyl di-*n*-pentylphosphinate as a golden oil: 3% yield; *R_{f}* = 0.33 (silica, 3:2, hexane: EtOAc); ¹H NMR (400 MHz, CDCl₃) *δ* 0.8 (t, J=7.2 Hz, 6H, (CH₂CH₂CH₂CH₂C*H*₃)₂), 1.29-1.39 (m, 8H, CH₂CH₂C*H*₂C*H*₂), 1.61-1.68 (m, 4H, PCH₂C*H*₂), 1.79-1.88 (m, 4H, PC*H*₂CH₂), 7.12-7.34 (m, 5H, Ph-H); ¹³C NMR (100MHz, CDCl₃) *δ* 13.76, 13.78, 21.53, 21.56, 22.11, 32.84, 32.99, 95.33, 96.08, 120.62, 120.67, 124.57, 129.50, 129.64, 129.75.

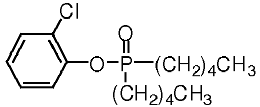

**2-Chlorophenyl di**-***n***-**pentylphosphinate**. Following the procedure above for the synthesis of phenyl dipentylphosphinate using 2-chlorophenyl dichlorophosphate, the crude product was purified by flash chromatography (silca gel, 3:2, hexane: EtOAc) to give 2-chlorophenyl di-*n*-pentylphosphinate as a golden oil: 11% yield; *R_{f}* = 0.33 (silica, 3:2, hexane:EtOAc); ¹H NMR (400 MHz, CDCl₃) *δ* 0.89 (t, J=7.6 Hz, 6H, (CH₂CH₂CH₂CH₂C*H*₃)₂), 1.28-1.41 (m, 8H, CH₂CH₂C*H*₂C*H*₂), 1.60-1.73 (m, 4H, PCH₂C*H*₂), 1.86-1.93 (m, 4H, PC*H*₂CH₂), 7.08 (t, J=7.7 Hz, 1H, Ar-H), 7.19-7.23 (m, 1H, Ar-H), 7.39 (d, J=7.9 Hz, 1H, Ar-H), 7.55 (dd, J=8.1, 1.0 Hz, 1H, Ar-H).

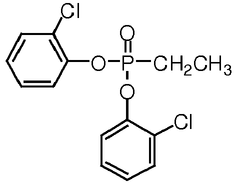

**Bis(2-chlorophenyl) ethylphosphonate.** A dried round bottom flask was charged with 0.60 mL (0.81 g, 5.5 mmol) of ethyl phosphonic dichloride and 9 mL of dry THF. To another dried round bottom flask sodium hydride (0.558 g of a 60% dispersion in mineral oil, approximately 14 mmol), 7 mL of dry THF and 2.1 equivalents (1.20 mL, 11.6 mmol) of 2-chlorophenol were added. The sodium 2-chlorophenoxide was added to the stirring phosphonic dichloride solution via cannulation. The mixture was allowed to react overnight at room temperature. The reaction mixture was dissolved in 50 mL of diethyl ether and washed three times with 10 mL of saturated sodium bicarbonate. The organic layer was then washed three times with 10 mL of saturated sodium chloride solution, dried over magnesium sulfate, filtered, and concentrated in vacuo to give 2.08 g of a pale yellow oil. The oil was purified by flash column chromatography (silica gel, 3:7, EtOAc:hexane) and evaporatively distilled (220°C/0.2 mm Hg) to afford bis(2-chlorophenyl) ethylphosphonate as a clear colorless oil: 28%; GCMS (*m*/*z*) 139 (100%), 295 (M⁺, 85%); *R_{f}*=0.41 (2:3, hexane:EtOAc); ¹H NMR (400 MHz, CDCl₃) *δ* 1.45 (dt,3H, J = 22.0, 7.6 Hz, CH₂C*H*₃), 2.26 (dq, 2H, J = 18.5, 7.7 Hz, C*H*₂CH₃), 7.09-7.14 (m, 1H, Ar-*H*), 7.17-7.22 (1H, Ar-*H*), 7.33 (dt, 1H, J = 8.2 Hz, 1.5 Hz, Ar-*H*), 7.42 (ddd, 1H, J = 8.0, 1.6, 0.8 Hz, Ar-*H*); ¹³C NMR (100 MHz, CDCl₃) *δ* 19.29, 20.70, 122.26, 122.29, 125.67, 125.73, 125.95, 125.96, 127.81, 127.83, 130.57, 146.22,146.30.

### Example 2: Cholinesterase Assays

Acetylcholinesterase (from electric eel), butyrylcholinesterase (from horse serum), DTNB, buyyrylthiocholine, acetylthiocholine, and BSA were purchased from Sigma. The dialkyl 2-chlorophenyl (and 4-chlorophenyl) phosphates were solubilized in reagent grade methanol.

Cholinesterase activity measurements were performed essentially according to the method of Ellman (Biochemical Pharmacology 7:88-90, 1961). The composition of the assay mixture was 0.1 M sodium phosphate, pH 7.5, 0.033 M DTNB, 0.001 M MgCl₂, and 100 µg/mL of BSA containing appropriate amounts of substrate and inhibitor. Each assay was initiated by the addition of substrate and the activity quantified by measuring the formation of the thionitrobenzoate anion at 412 nm at 37°C. Methanol used to solubilize the dialkyl phenyl phosphates [1% (v/v) final concentration] had no inhibitory effect on either enzyme.

Butyrylcholinesterase was pre-incubated at room temperature with inhibitor in the assay cocktail minus substrate for varying periods of time at 25°C. Substrate was then added and the activity measured as described above. The relative rates of enzyme inactivation were determined by plotting enzyme activity versus time of pre-incubation with inhibitor. Results were expressed as the rate constant for the rate of enzyme inactivation. In the case of AcChase, results were expressed as the % of activity remaining relative to the enzyme incubated only with solvents.

Enzyme activity was also measured by native gel electrophoresis. For cholinesterase activity, proteins (10 µg of both acetylcholinesterase and butyrylcholinesterase) were fractionated on Novex precast 10% Tris glycine gels (Invitrogen). Enzyme activity was detected by first incubating the gels in a solution of 5 mM substrate then staining with a solution of copper sulfate/potassium ferricyanide.

The first series of experiments determined the effect of the dialkyl 2-chlorophenyl (and 4-chlorophenyl) phosphates on both AcChase and BuChase. None of the compounds had any inhibitory effect on AcChase (See Table 1). In Table 1, AcChase was incubated with 100 µM of the indicated phenyl phosphate for 60 minutes at 25°C and then assayed as described above. Results were expressed as the % activity relative to enzyme incubated with solvent ± SD (standard deviation).

**Table 1. Effect of dialkyl 2-chlorophenyl phosphates on acetylcholinesterase activity.**

| **Compound** | **% Activity ± SD** |
|---|---|
| Di-methyl- | 101.9 ± 0.019 |
| Di-ethyl- | 96.77 ± 0.019 |
| Di-*n*-propyl- | 97.38 ± 0.028 |
| Di-*iso*-propyl- | 97.82 ± 0.045 |
| Di-*n*-butyl- | 97.60 ± 0.029 |
| Di-*iso*-butyl- | 96.18 ± 0.023 |
| Di-*n*-butyl-(4Cl) | 97.61 ± 0.006 |
| Di-*n*-pentyl- | 101.7 ± 0.030 |

In contrast, several of the compounds showed inhibitory activity on BuChase. While di-methyl- and di-isopropyl 2-chloropheynyl phosphates appeared to have no effect on BuChase activity under standard conditions, they showed inhibitory activity when their concentration was increase as shown in Table 2. The remaining derivatives showed significant inhibitory activity against the enzyme under standard conditions (Table 2). Interestingly, the degree of inhibitory activity, measured as the rate of enzyme inactivation, appears to be a function of the structure of the derivative. In Table 2, BuChase was incubated for increasing periods of time at 25°C with 100 nM inhibitor then assayed for activity as described above. Rate constants were calculated from a plot of enzyme activity vs. time of inhibitor exposure. Activity measurements at each time point (3 to 4 per compound) were performed in triplicate. Slopes were calculated by linear regression analysis.

**Table 2. Rates of inactivation of butyrylcholinesterase by di-alkyl 2-chlorophenyl phosphates.**

| | 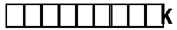 |
|---|---|
| **Compound: (di-alkyl 2-chlorophenyl phosphate)** | **(min⁻¹) x 10⁻² ± S.E.M / Comments** |
| Di-methyl- | Inhibitory at 10⁻⁴ M, 48.9% Inhibition |
| Di-isopropyl- | Inhibitory at 10⁻⁴ M, 85.3% Inhibition |
| Di-ethyl- | 6.64 ± 2.1 |
| Di-n-prop- | 20.21 ± 2.2 |
| Di-n-butyl- | 64.76 ± 3.0 |
| Di-isobutyl- | 60.83 ± 5.8 |
| Di-n-butyl- (4Cl) | 13.01 ± 2.0 |
| Di-n-pentyl- | 30.44 ± 3.3 |
| Control | 1.64 ± 1.1 |

Exhaustive dialysis of the inhibited enzyme did not restore enzyme activity, suggesting that the enzyme had been covalently modified, i.e., phosphorylation of the serine within the enzyme's catalytic triad. In some cases of inhibition, cholinesterases are capable of regaining catalytic activity.

Table 3 depicts reactivation of inactive butyrylcholinesterase. BuChase was incubated with excess di-butyl 2-chlorophenyl phosphate for 60 min at 4°C. The excess phosphate was removed using a desalting column. The inactivated BuChase was incubated at 4°C for various periods of time and then assayed for activity. Results were expressed as percent inhibition and calculated using the following formula; [(activity with solvent only) - (activity with DB-2Cl-PP)] / (activity with solvent only) x 100 %. There is no significant reactivation over 24 hours as the % remaining activities are statistically the same.

**Table 3. Reactivation of inactive butyrylcholinesterase**

| Incubation Period | 0 hours | 3.5 hours | 7 hours | 24 hours |
|---|---|---|---|---|
| % Remaining Activity | 0.6 | 0.6 | 0.8 | 1.1 |

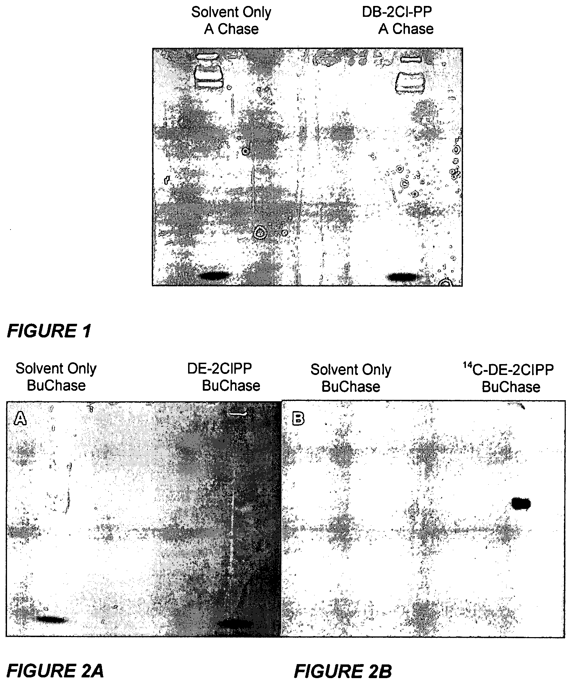

Using native gel electrophoresis, both AcChase and BuChase activity was detected. Pre-incubation of AcChase with di-ethyl 2-chlorophenyl phosphate had no effect on AcChase activity (Figure 1). In contrast, the activity of BuChase was nearly completely inhibited by di-ethyl 2-chlorophenyl phosphate (Figure 2A).

The cholinesterases were pre-incubated with ¹⁴C-di-ethyl-2-chlorophenyl phosphate and then fractionated by native gel electrophoresis as described above. The proteins were fixed in the gel with 10% acetic acid/30% methanol. The fixative was removed and replaced with 100 mL of EN³HANCE. The gel was incubated for 60 minutes at room temperature with gentle agitation and then placed in 5% PEG and incubated for 30 minutes at room temperature with gentle agitation. The gel was then dried down on a piece of 3MM filter paper, exposed to X-ray film at -80°C for 2 weeks, and then developed. Pre-incubation of BuChase with ¹⁴C-labeled di-ethyl 2-chlorophenyl phosphate and gel electrophoresis of the mixture showed that the compound had been covalently modified by the compound (Figure 2B).

Tables 4 below shows the relative inhibitory activity of exemplary compounds of the present disclosure. The results were obtained by pre-incubating the enzyme with the compound (final concentration 10⁻⁷M) for various periods of time and measuring the remaining enzyme activity. The results are shown as the change in absorbance at 412 nm / minute. In order to simplify the results from all the compounds, the enzyme activities were normalized to 2-chlorophenyl-di-n-butyl phosphate which was set as 1.0. Since two different preparations of enzyme were used, the normalization procedure would adjust for any differences in the amount of enzyme. Several of the compounds are potent inhibitors, i.e., the rates of inactivation are so fast that they were not measurable under these conditions. In other cases, the compound's inhibitory activity is not as strong and assay conditions were modified to increase the inhibitor concentration.

**Table 4: Rates of inactivation of butyrylcholinesterase by exemplary compounds: Change in Absorbance at 412nm / Minute. (ND = No Data)**

| **Compounds** | **Change in Absorbance at 412nm /Minute (Not Normalized) / Comments** | **Change in Absorbance at 412nm /Minute (Normalized)** |
|---|---|---|
| 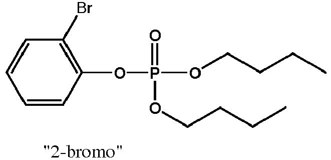 | 0.918 | 1.366 |
| 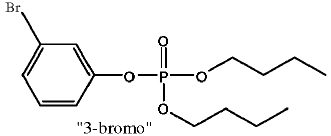 | 4.18 | 6.220 |
| 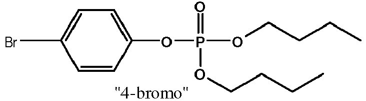 | 0.495 | 0.737 |
| 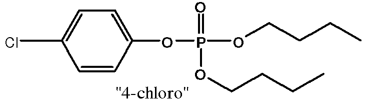 | 0.227 | 0.338 |
| 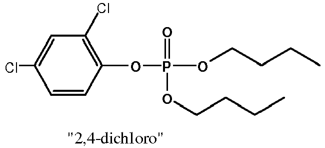 | 92% inhibition after 1 minute under standard conditions | |
| 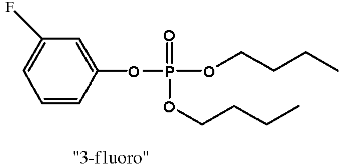 | 0.468 | 0.696 |
| 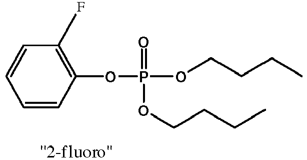 | 1.75 | 2.604 |
| 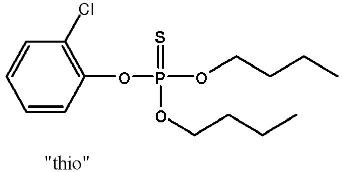 | Not Inhibitory under standard conditions/ Inhibitory at 10⁻⁴ M. 14.6% inhibition | |

| **Compounds** | **Change in Absorbance at 412nm /Minute (Not Normalized) / Comments** | **Change in Absorbance at 412nm /minute (Normalized)** |
|---|---|---|
| 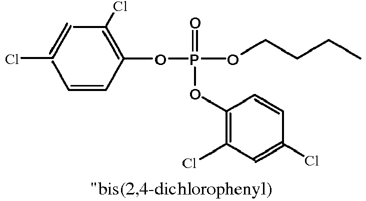 | Not inhibitory under standard conditions. Inhibitory at 10⁻⁴ M: 63.4% inhibition. | |
| 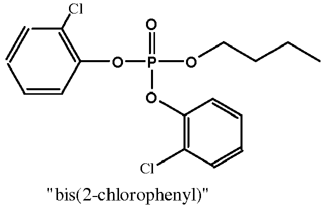 | ND | ND |
| 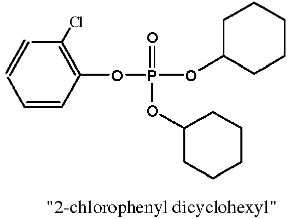 | ND | ND |
| 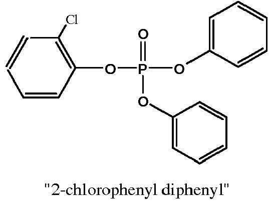 | Not inhibitory under standard conditions. Inhibitory a 10⁻⁴ M: 29.4% inhibition | |
| 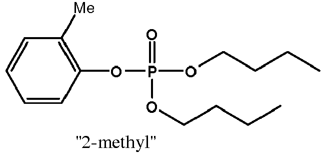 | Reversible inhibitor: Kᵢ = 2.1 µM | |
| 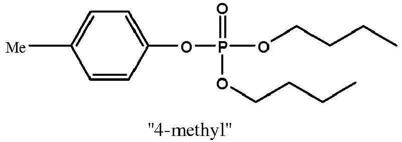 | ND | ND |
| 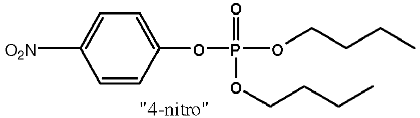 | 100% Inhibition (Immediate under standard conditions) | |
| 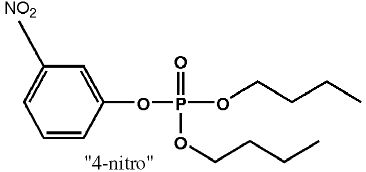 | 83% Inhibition (Immediate). 100% Inhibition after 2 minutes | |
| 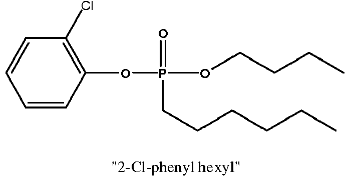 | 10.04 | 14.94 |
| 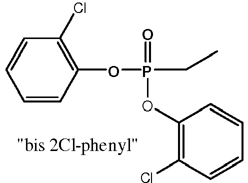 | 0.688 | 1.024 |
| 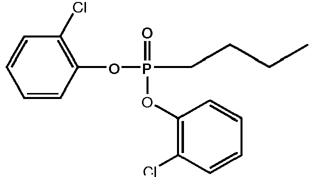 | 0.32 | 0.48 |
| 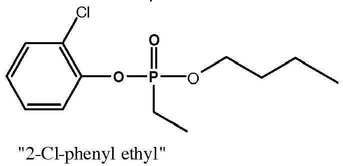 | ND | ND |
| 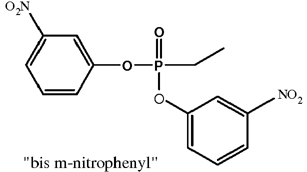 | ND | ND |
| 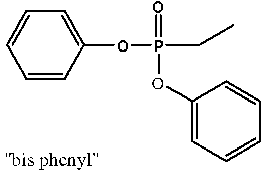 | ND | ND |
| 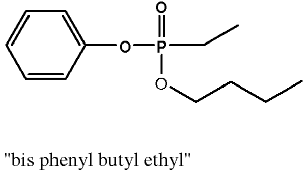 | ND | ND |
| 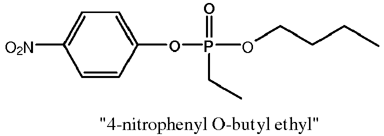 | 100% inhibition under standard conditions | ND |
| 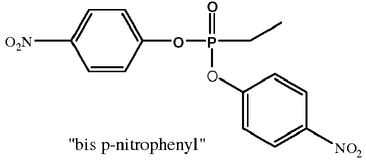 | ND | ND |
| 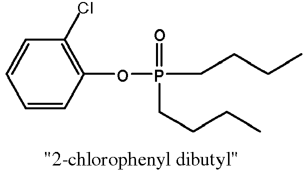 | 0.684 | 1.018 |
| 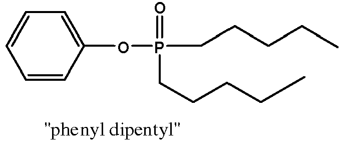 | ND | ND |
| 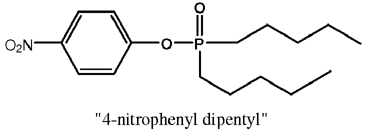 | ND | ND |
| 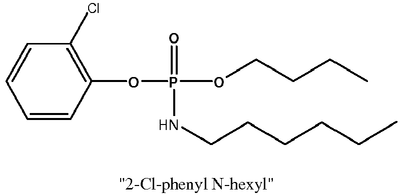 | 0.688 | 1.024 |
| 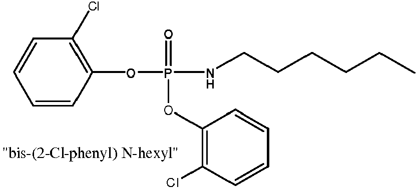 | Not inhibitory under standard conditions. Inhibitory a 10⁻⁴ M: 73.9% inhibition. | |

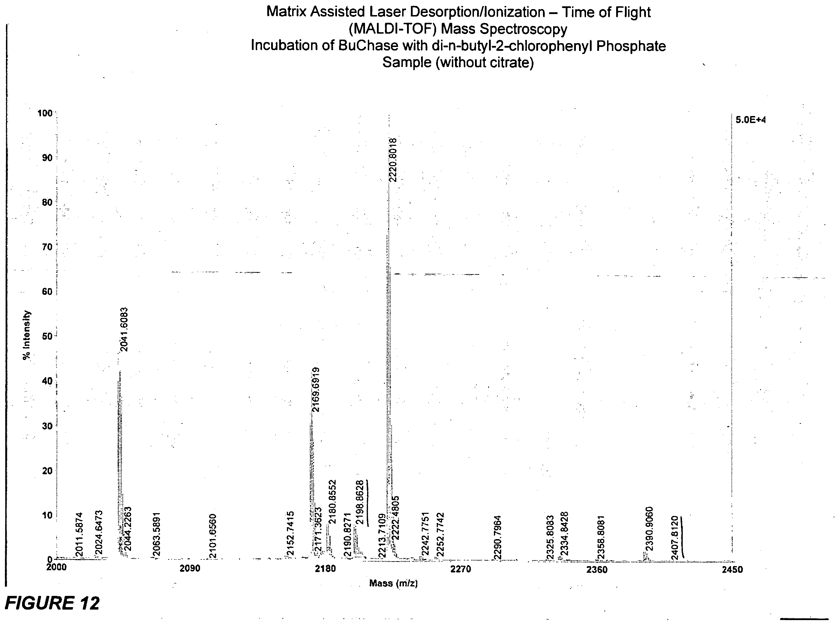

Matrix Assisted Laser Desorption/Ionization Time of Flight (MALDI-TOF) Mass Spectroscopy was used to show that BuChase was covalently modified (Figure 12). BuChase was incubated with di-n-butyl-2-chlorophenyl phosphate. Excess inhibitor was removed and the enzyme was digested with trypsin. The tryptic peptides were then analyzed by MALDI-TOF. Unmodified peptide containing the active site serine (MW 2198.86) and modified peptide (MW 2390.9060) can be seen. This is what would be predicted if BuChase had been covalently modified. Irreversible inhibitors usually covalently modify an enzyme, and inhibition cannot therefore be reversed. This results shows that di-n-butyl-2-chlorophenyl phosphate is likely an irreversible inhibitor.

Many of the presently disclosed compounds are irreversible inhibitors. In some respects, it may be desirable to have irreversible inhibitors which act on butyrylcholinesterase as there may be such advantages as reduced dosage and greater efficacy, etc.

### Example 3: Specificity of Butyrylcholinesterase Inhibitors

The specificity of di-n-butyl 2-chlorophenyl phosphate (DB-2Cl-PP) was determined by its effects on the serine proteases trypsin and chymotrypsin, protein kinases Protein Kinase A and S6K2, and hexokinase.

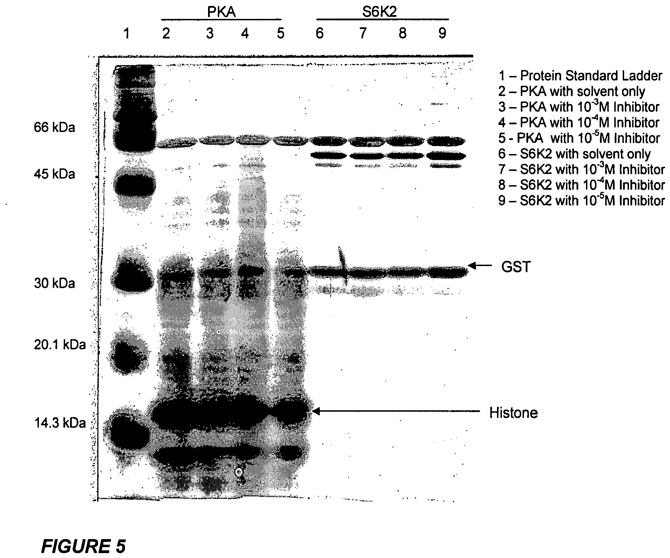

For protein kinase activity, histone was used as the substrate for protein kinase A (PKA) and glutathione-S-transferase-S6 (GST-S6) for S6K2. Enzyme was pre-incubated with substrate and varying concentrations of inhibitor on ice for 30 minutes. The control sample contained solvent (methanol). ATP was then added to each sample and the cocktail incubated at 30°C for 15 minutes. Equal amounts of each mixture were fractionated by SDS-PAGE on a 10% acrylamide gel. The gel was fixed in 50% methanol/10% acetic acid, washed with water, and then stained in Pro-Q Stain in the dark for 60 minutes. The gel was then destained, washed, and scanned on a Typhoon Imager (Figure 5).

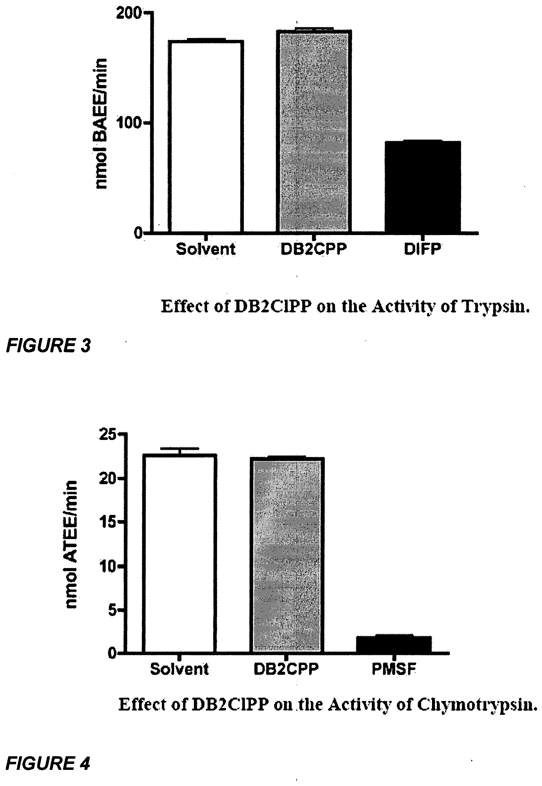

The effect of DB2CIPP (di-n-butyl 2-chlorophenyl phosphate) on the activity of trypsin was assayed. Trypsin was incubated with solvent, 10 µM DB2CPP, or 10 µM DIFP (a known inhibitor of trypsin) for 60 minutes at 25°C to determine any inhibitory effect of DB2CPP on enzymatic activity. Enzyme activity is given as nmoles of BAEE hydrolyzed per minute at 25°C. Data shown are the mean of three replicates ± SEM. (Figure 3)

The effect of DB2CIPP on the activity of chymotrypsin was assayed. Trypsin was incubated with solvent, 10 µM DB2CPP, or 10 µM PMSF (a known inhibitor of chymotrypsin) for 60 minutes at 25°C to determine any inhibitory effect of DB2CPP on enzymatic activity. Enzyme activity is given as nmoles of ATEE hydrolyzed per minute at 25°C. Data shown are the mean of three replicates ± SEM. (Figure 4)

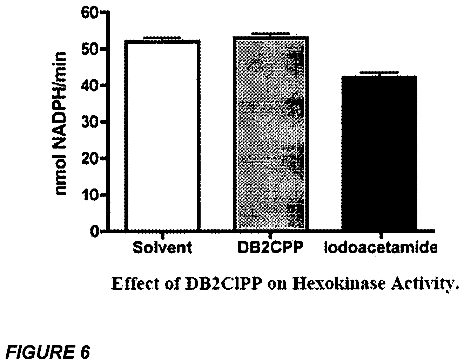

The effect of DB2CIPP on hexokinase activity was assayed. Hexokinase was incubated with solvent, 10 mM iodoacetamide (a known hexokinase inhibitor), or 10 µM DB2CPP for 60 min at 25°C. Enzyme activity is given as the nmoles of NADPH generated per min at 25°C. Data shown are the mean of three replicates ± SEM. (Figure 6)

As seen in Figures 3 (trypsin), 4 (chymotrypsin), 5 (PKA and S6K2), and 6 (hexokinase), none of the above mentioned enzymes were affected by di-n-butyl 2-chlorophenyl phosphate.

### Example 4: Effects of Butyrylcholinesterase Inhibitors on Cells

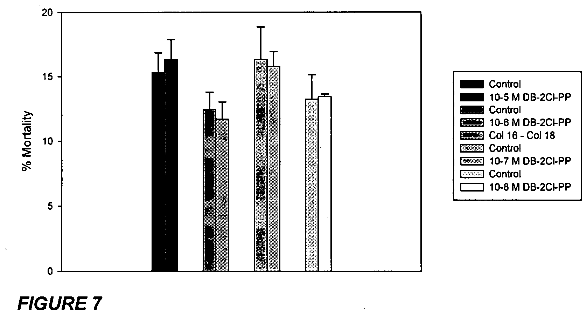

Porcine umbilical stem cells were cultured in Neurobasal Medium supplemented with B27 (Invitrogen) and antibiotics for various periods of with varying concentrations of din-butyl-2-chlorophenyl phosphate. Cells were collected, washed, counted, and analyzed for viability by Trypan Blue staining. Di-n-butyl-2-chlorophenyl phosphate did not have any significant effect on mortality of the porcine umbilical stem cells (Figure 7).

In addition, uptake of di-n-butyl-2-chlorophenyl phosphate by porcine umbilical stem cells was measured after 24 and 48 hours of culture (Table 5).

**Table 5**

| Incubation Period | 0 hours | 24 hours | 48 hours |
|---|---|---|---|
| fg DB2Cl-PP /cell (± SEM) | 2.55 ± 0.011 | 11.57 ± 1.02 | 13.62 ± 0.003 |

Also, the uptake of di-n-butyl-2-chlorophenyl phosphate by human umbilical cells was measured after 24 of culture for both undifferentiated cells and differentiating neurons. (Table 6)

**Table 6**

| | 0 hours | 24 hours |
|---|---|---|
| Undifferentiated | fg/cell | fg/cell |
| | 114.72±37.93 | 795.89 ±202.4112 |
| | | |

| | 0 hours | 24 hours |
|---|---|---|
| Neurons | fg/cell | fg/cell |
| | 78.85 ±8.04 | 820.52±82.32 |

### Example 5: Modeling of Flexible Docking of Inhibitor with AcChase and BuChase

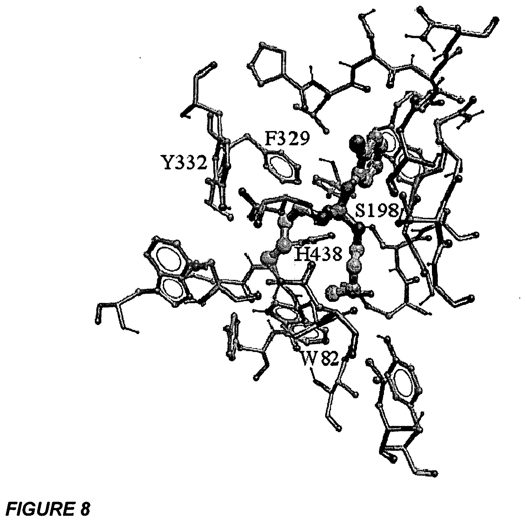

Modeling of a library of dialkyl phenyl phosphates and flexible docking with AcChase and BuChase were accomplished using ICM-Pro. ICM PocketFinder was used to identify and define the receptor target region for docking. The enzyme structures used for docking were human AcChase (PDB ID: 1B41) and BuChase (PDB ID: 1P0l). The result for one compound in the library is shown as a stick rendering in Figure 8. Shown is best ranked pose by binding energy. Protein side chains are rendered as thin sticks. Ligand is shown in ball and stick representation, with sticks widened for emphasis. Only polar hydrogens are shown for clarity. Known active site residues are identified.

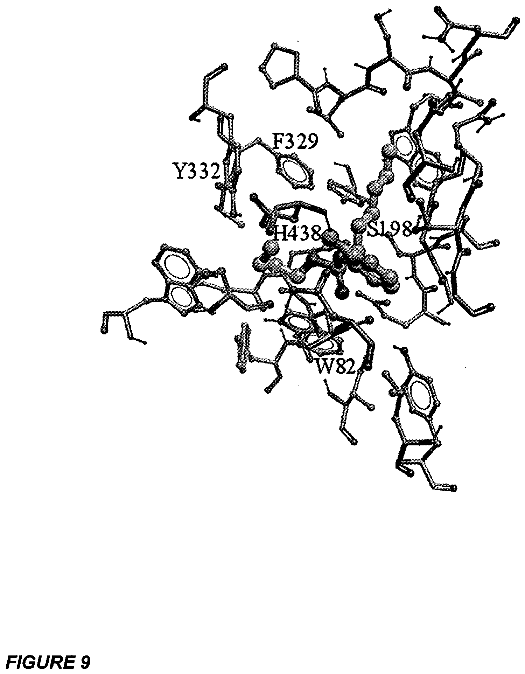

Modeling of a library of dialkyl phenyl and diphenyl alkyl phosphonates, and flexible docking with AcChase and BuChase were accomplished using ICM-Pro. ICM PocketFinder was used to identify and define the receptor target region Modeling of a library of dialkyl phenyl phosphates and flexible docking with AcChase and BuChase were accomplished using ICM-Pro. ICM PocketFinder was used to identify and define the receptor target region for docking. The enzyme structures used for docking were human AcChase (PDB ID: 1B41) and BuChase (PDB ID: 1P0l). The result for one compound in the library is shown as a stick rendering in Figure 9. Shown is best ranked pose by binding energy. Protein side chains are rendered as thin sticks. Ligand is shown in ball and stick representation, with sticks widened for emphasis. Only polar hydrogens are shown for clarity. Known active site residues are identified.

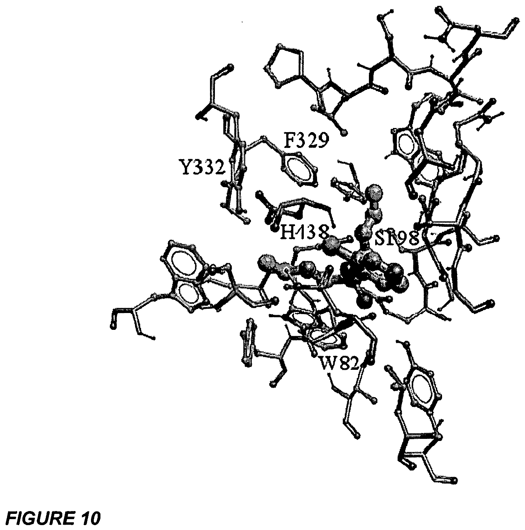

Modeling of a library of dialkyl phenyl phosphinates and flexible docking with AcChase and BuChase were accomplished using ICM-Pro. ICM PocketFinder was used to identify and define the receptor target region for docking. The enzyme structures used for docking were human AcChase (PDB ID: 1B41) and BuChase (PDB ID: 1P0l). The result for one compound in the library is shown as a stick rendering in Figure 10. Shown is best ranked pose by binding energy. Protein side chains are rendered as thin sticks. Ligand is shown in ball and stick representation, with sticks widened for emphasis. Only polar hydrogens are shown for clarity. Known active site residues are identified.

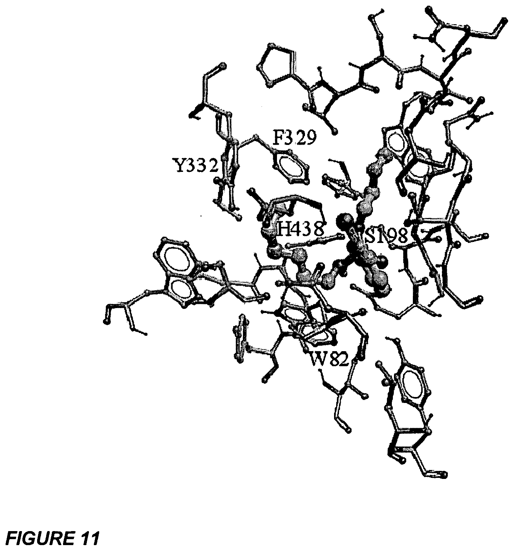

Modeling of a library of dialkyl phenyl and diphenyl alkyl phosphoramidates, and flexible docking with AcChase and BuChase were accomplished using ICM-Pro. ICM PocketFinder was used to identify and define the receptor target region for docking. The enzyme structures used for docking were human AcChase (PDB ID: 1B41) and BuChase (PDB ID: 1P0I). The result for one compound in the library is shown as a stick rendering in Figure 11. Shown is best ranked pose by binding energy. Protein side chains are rendered as thin sticks. Ligand is shown in ball and stick representation, with sticks widened for emphasis. Only polar hydrogens are shown for clarity. Known active site residues are identified.

Structural details provided by the modeling studies validate the kinetic data shown in the present disclosure. This in silico docking-guided semi-rational approach has shown to be a very valuable methodology for elucidating structural requirements necessary for inhibitors of exceptionally high BuChase selectivities, thus assisting in the development of BuChase-selective compounds for the treatment of AD.

## Claims

1. Use of a compound in the manufacture of a medicament to inhibit butyrylcholinesterase, wherein said compound has the structure of one of Formulas 1 and 2: 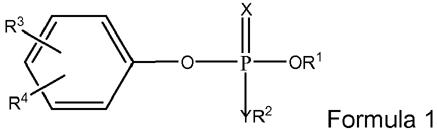 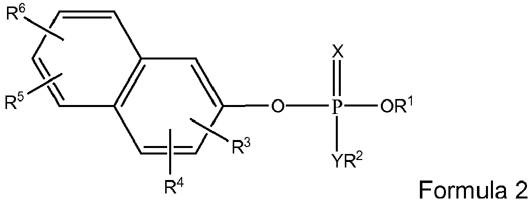 wherein
X is O or S;
Y is O or N;
R¹ and R² can be the same or different and are independently selected from the group consisting of H, C₁₋₆ alkyl, C₁₋₆ alkenyl, and unsubstituted or substituted phenyl;
wherein the potential substituents of the substituted phenyl group are selected from F, Cl, Br, I, or NO₂;
R³ and R⁴ in formula I can be the same or different and are independently selected from the group consisting of H, methyl, methoxy, F, Cl, Br and I; and
R³ to R⁶ in formula II can be the same or different and are independently selected from the group consisting of H, methyl, methoxy, F, Cl, Br, I and NO₂;
or a pharmaceutically acceptable salt thereof.

2. Use according to claim 1, wherein said phenyl is substituted at least once wherein each substituent is independently selected from the group consisting of H, methyl, methoxy, F, Cl, Br or I in formula I; and said phenyl is substituted at least once wherein each substituent is independently selected from the group consisting of H, methyl, methoxy, F, Cl, Br, I and NO₂ in formula II.

3. Use according to claim 1, wherein said C₁₋₆ alkyl is selected from the group consisting of methyl, ethyl, n-propyl, isopropyl, n-butyl, isobutyl, cyclohexyl, and n-pentyl.

4. Use according to claim 1, wherein said compound is selected from the group consisting of: 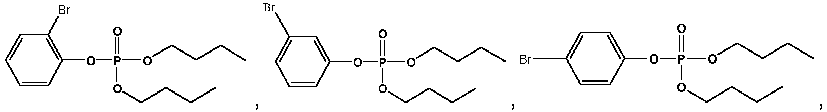 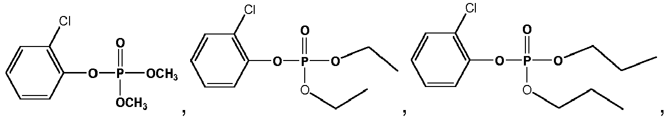 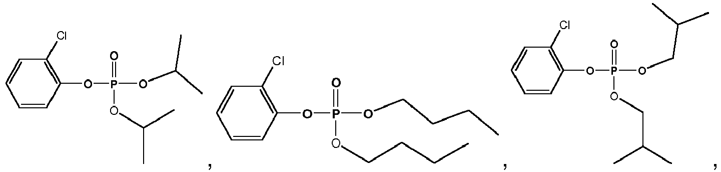 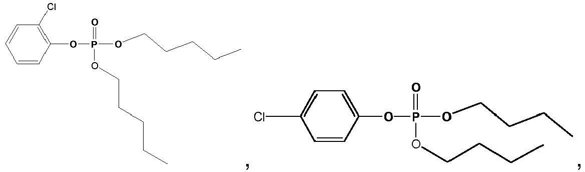 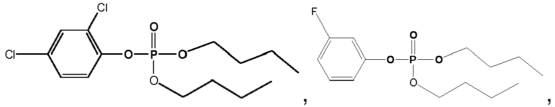 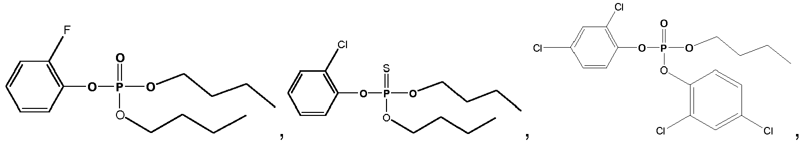 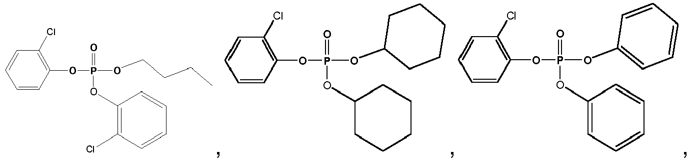 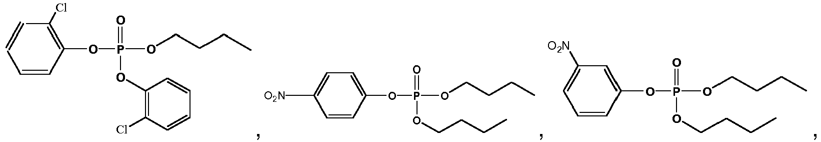 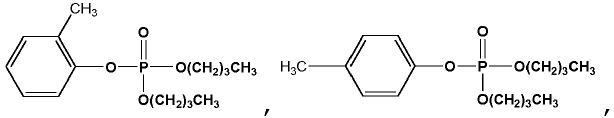 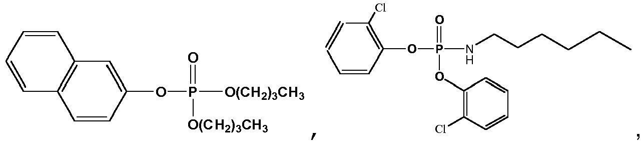 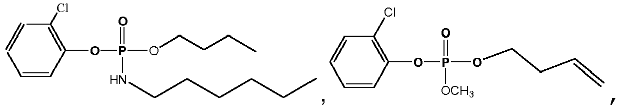 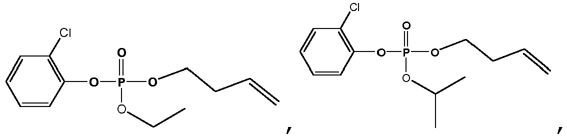 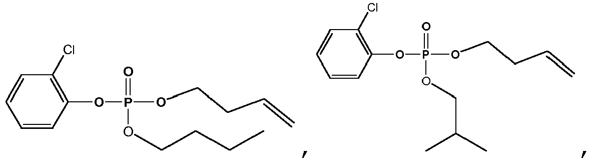 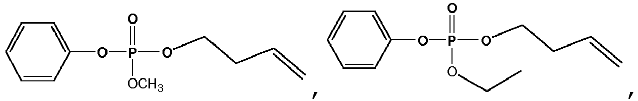 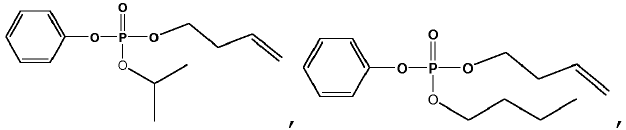 and

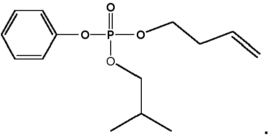

5. Use of a compound in the manufacture of a medicament for the treatment of a neurodegenerative disease, wherein said compound has the structure of Formulas 1 or 2: 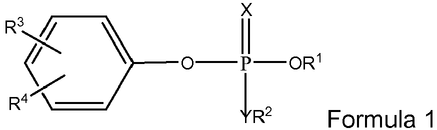 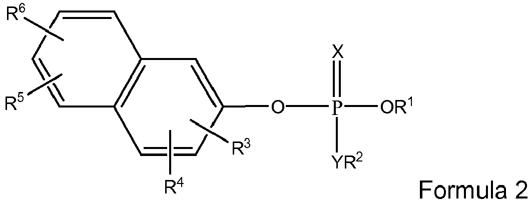 wherein
X is O or S;
Y is O or N;
R¹ and R² can be the same or different and are independently selected from the group consisting of H, C₁₋₆ alkyl, C₁₋₆alkenyl, and unsubstituted or substituted phenyl;
wherein the potential substituents of the substituted phenyl group are selected from F, Cl, Br, I, or NO₂;
R³ and R⁴ in formula I can be the same or different and are independently selected from the group consisting of H, methyl, methoxy, F, Cl, Br and I; and
R³ to R⁶ in formula II can be the same or different and are independently selected from the group consisting of H, methyl, methoxy, F, Cl, Br, I and NO₂;
or a pharmaceutically acceptable salt thereof; and
inhibiting butyrylcholinesterase.

6. The use of claim 5, wherein said phenyl is substituted at least once wherein each substituent is independently selected from the group consisting of H, methyl, methoxy, F, Cl, Br or I in formula I; and said phenyl is substituted at least once wherein each substituent is independently selected from the group consisting of H, methyl, methoxy, F, Cl, Br, I and NO₂ in formula II.

7. The use of claim 5, wherein said C₁₋₆ alkyl is selected from the group consisting of methyl, ethyl, n-propyl, isopropyl, n-butyl, isobutyl, cyclohexyl, and n-pentyl.

8. The use of claim 5, wherein said compound is selected from the group consisting of: 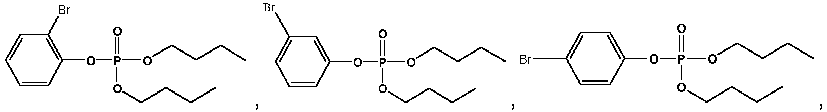 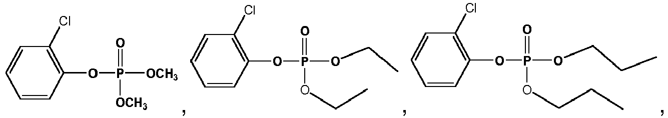 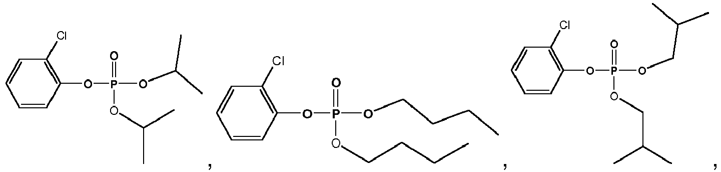 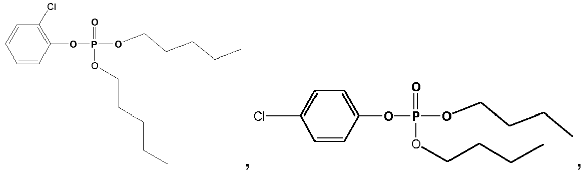 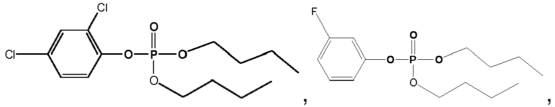 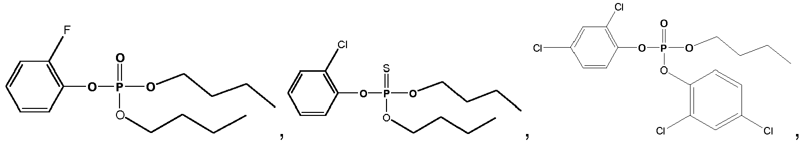 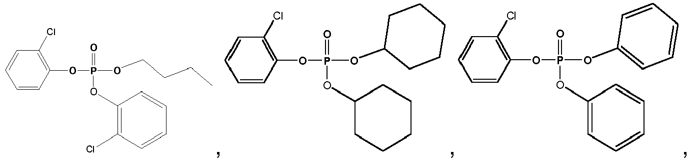 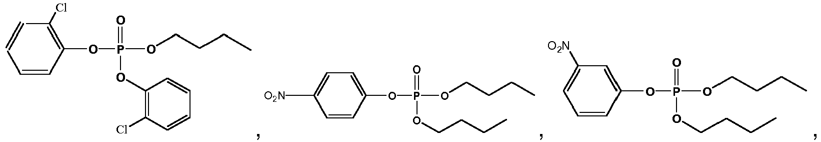 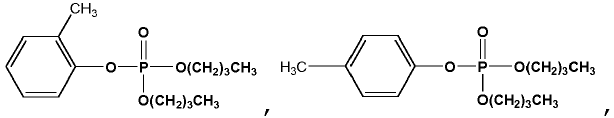 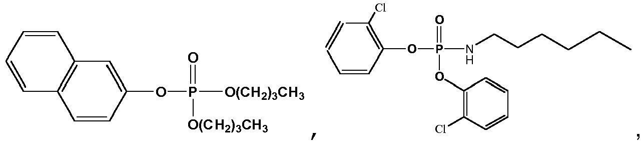 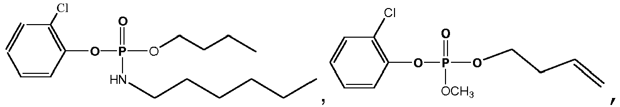 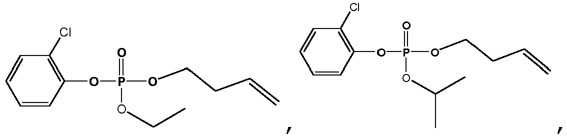 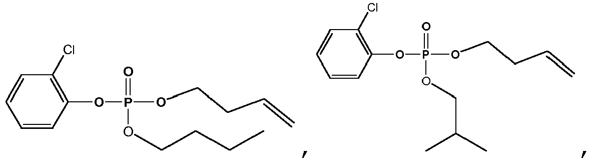 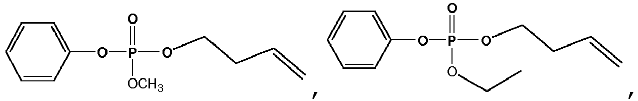 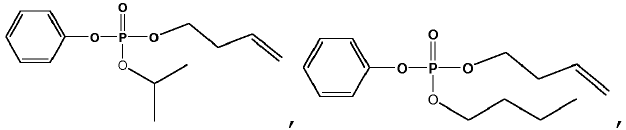 and

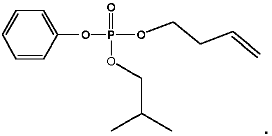

9. The use according to claim 5 wherein said neurodegenerative disease is Alzheimer's disease.

## Patentansprüche

1. Verwendung einer Verbindung bei der Herstellung eines Arzneimittels zum Hemmen von Pseudocholinesterase, wobei die Verbindung die Struktur einer der Formeln 1 und 2 hat: 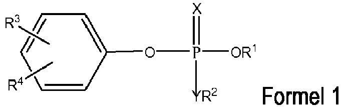 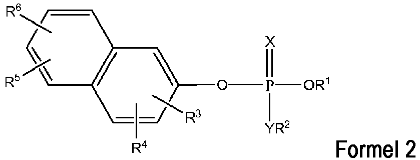 wobei
X O oder S ist;
Y O oder N ist;
R¹ und R² gleich oder verschieden sein können und unabhängig voneinander aus der Gruppe, bestehend aus H, C₁₋₆-Alkyl, C₁₋₆-Alkenyl und unsubstituiertem oder substituiertem Phenyl, ausgewählt sind;
wobei die potentiellen Substituenten der substituierten Phenylgruppe aus F, Cl, Br, I oder NO₂ ausgewählt sind;
R³ und R⁴ in Formel I gleich oder verschieden sein können und unabhängig aus der Gruppe, bestehend aus H, Methyl, Methoxy, F, Cl, Br und I, ausgewählt sind; und
R³ bis R⁶ in Formel II gleich oder verschieden sein können und unabhängig aus der Gruppe, bestehend aus H, Methyl, Methoxy, F, Cl, Br und NO₂, ausgewählt sind;
oder ein pharmazeutisch verträgliches Salz davon.

2. Verwendung nach Anspruch 1, wobei das Phenyl mindestens einmal substituiert ist, wobei jeder Substituent unabhängig aus der Gruppe, bestehend aus H, Methyl, Methoxy, F, Cl, Br oder I in Formel I, ausgewählt ist; und das Phenyl mindestens einmal substituiert ist, wobei jeder Substituent unabhängig aus der Gruppe, bestehend aus H, Methyl, Methoxy, F, Cl, Br, I und NO₂ in Formel II, ausgewählt ist.

3. Verwendung nach Anspruch 1, wobei das C₁₋₆-Alkyl aus der Gruppe, bestehend aus Methyl, Ethyl, n-Propyl, Isopropyl, n-Butyl, Isobutyl, Cyclohexyl und n-Pentyl, ausgewählt ist.

4. Verwendung nach Anspruch 1, wobei die Verbindung aus der Gruppe ausgewählt ist, die aus Folgendem besteht: 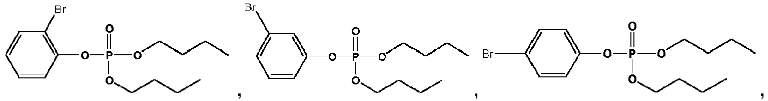 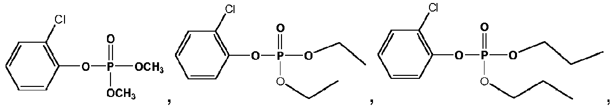 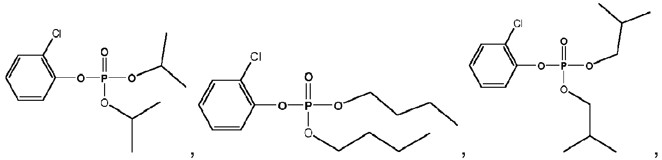 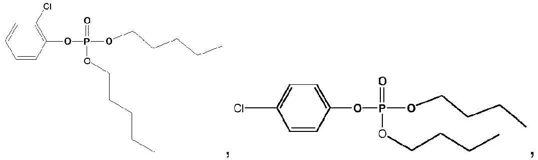 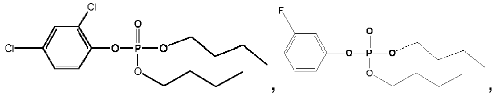 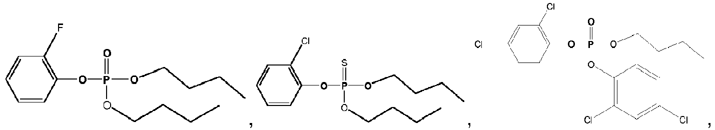 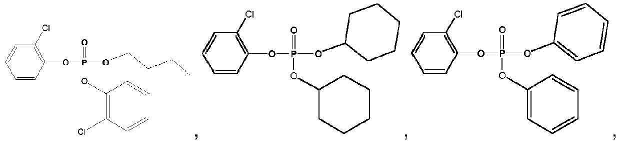 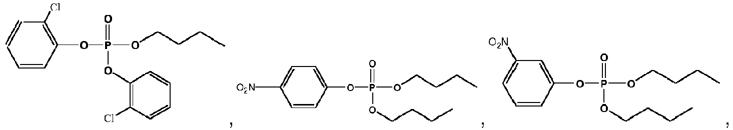 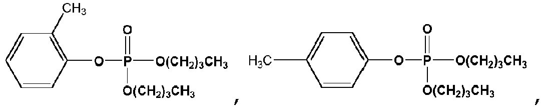 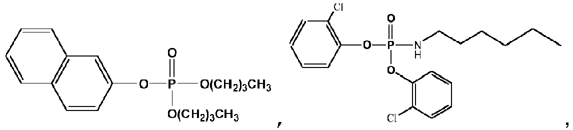 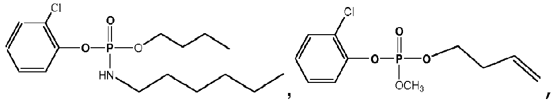 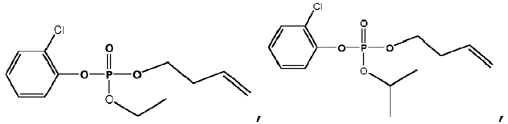 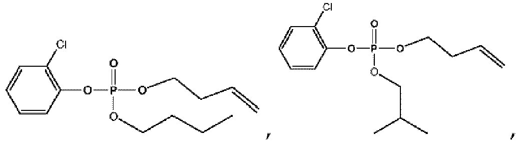 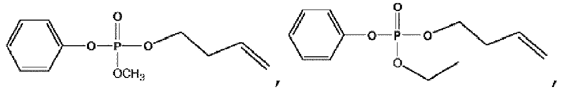 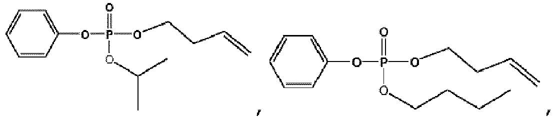 und

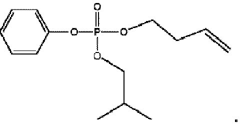

5. Verwendung einer Verbindung bei der Herstellung eines Arzneimittels zur Behandlung einer neurodegenerativen Erkrankung, wobei die Verbindung die Struktur von Formel 1 oder 2 aufweist: 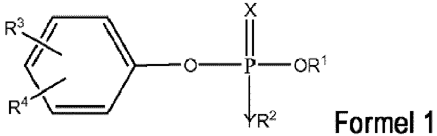 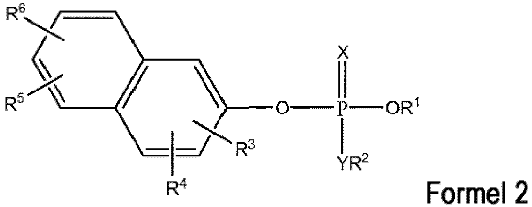 wobei
X O oder S ist;
Y O oder N ist;
R¹ und R² gleich oder verschieden sein können und unabhängig voneinander aus der Gruppe, bestehend aus H, C₁₋₆-Alkyl, C₁₋₆-Alkenyl und unsubstituiertem oder substituiertem Phenyl, ausgewählt sind;
wobei die potentiellen Substituenten der substituierten Phenylgruppe aus F, Cl, Br, I oder NO₂ ausgewählt sind;
R³ und R⁴ in Formel I gleich oder verschieden sein können und unabhängig von der Gruppe, bestehend aus H, Methyl, Methoxy, F, Cl, Br und I, ausgewählt sind; und
R³ bis R⁶ in Formel II gleich oder verschieden sein können und unabhängig von der Gruppe, bestehend aus H, Methyl, Methoxy, F, Cl, Br, I und NO₂, ausgewählt sind;
oder ein pharmazeutisch verträgliches Salz davon; und
wobei Pseudocholinesterase gehemmt wird.

6. Verwendung von Anspruch 5, wobei das Phenyl mindestens einmal substituiert ist, wobei jeder Substituent unabhängig aus der Gruppe, bestehend aus H, Methyl, Methoxy, F, Cl, Br oder I in Formel I, ausgewählt ist; und das Phenyl mindestens einmal substituiert ist, wobei jeder Substituent unabhängig aus der Gruppe, bestehend aus H, Methyl, Methoxy, F, Cl, Br, I und NO₂ in Formel II, ausgewählt ist.

7. Verwendung nach Anspruch 5, wobei das C₁₋₆-Alkyl aus der Gruppe, bestehend aus Methyl, Ethyl, n-Propyl, Isopropyl, n-Butyl, Isobutyl, Cyclohexyl und n-Pentyl, ausgewählt ist.

8. Verwendung nach Anspruch 5, wobei die Verbindung aus der Gruppe ausgewählt ist, die aus Folgendem besteht: 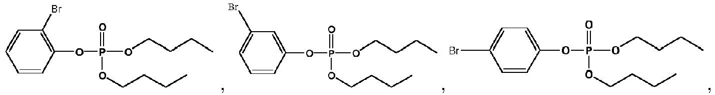 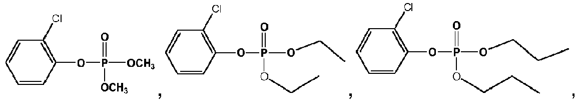 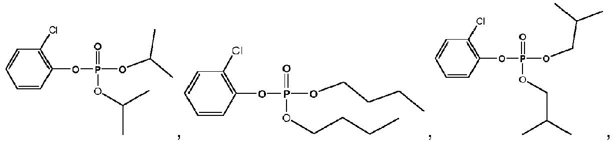 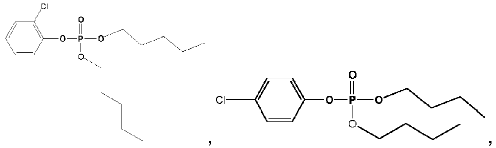 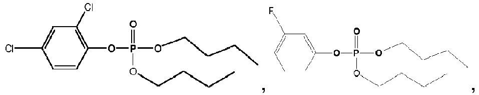 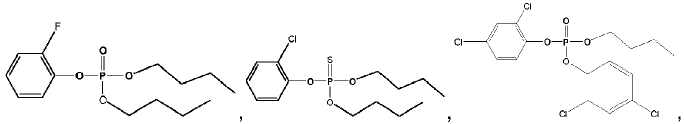 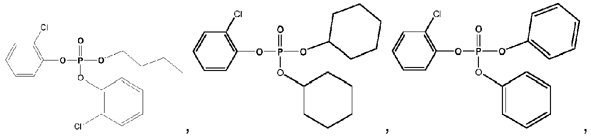 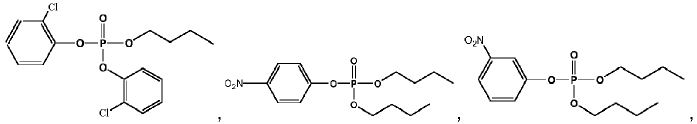 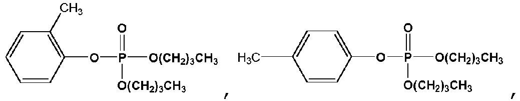 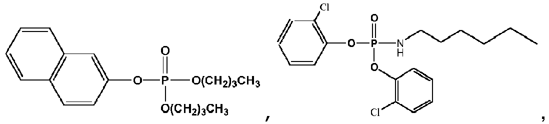 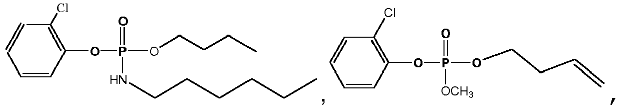 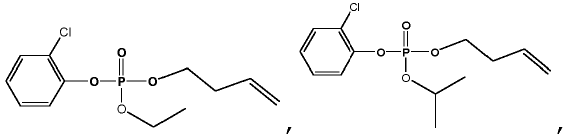 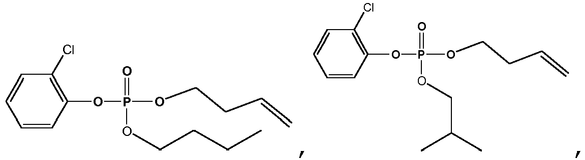 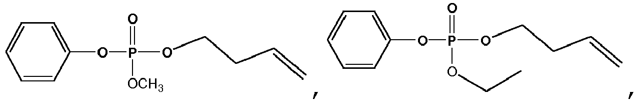 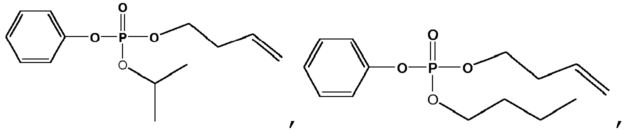 und

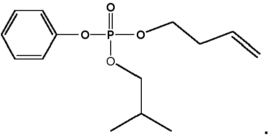

9. Verwendung nach Anspruch 5, wobei die neurodegenerative Erkrankung die Alzheimer-Krankheit ist.

## Revendications

1. Utilisation d'un composé dans la fabrication d'un médicament pour inhiber le butyrylcholinestérase, dans laquelle ledit composé a la structure de l'une des Formules 1 et 2 : 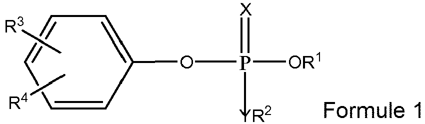 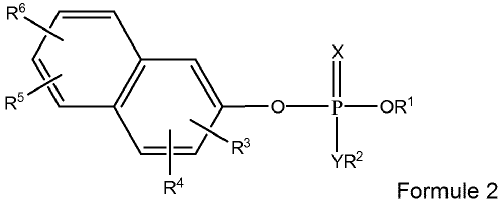 dans laquelle
X est O ou S ;
Y est O ou N ;
R¹ et R² peuvent être identiques ou différents et sont indépendamment choisis dans le groupe consistant en H, alkyle en C₁₋₆, alcényle en C₁₋₆, et phényle non substitué ou substitué ;
dans laquelle les substituants potentiels du groupe de phényle substitué sont choisis parmi F, CI, Br, I, ou NO₂;
R³ et R⁴ en formule I peuvent être identiques ou différents et sont indépendamment choisis dans le groupe consistant en H, méthyle, méthoxy, F, CI, Br et I ; et
R³ à R⁶ en formule II peuvent être identiques ou différents et sont indépendamment choisis dans le groupe consistant en H, méthyle, méthoxy, F, CI, Br, I, et NO₂ ;
ou un sel pharmaceutiquement acceptable de celui-ci.

2. Utilisation selon la revendication 1, dans laquelle ledit phényle est substitué au moins une fois dans laquelle chaque substituant est indépendamment choisi dans le groupe consistant en H, méthyle, méthoxy, F, CI, Br ou I en formule I ; et ledit phényle est substitué au moins une fois dans laquelle chaque substituant est indépendamment choisi dans le groupe consistant en H, méthyle, méthoxy, F, CI, Br, I et NO₂ en formule II.

3. Utilisation selon la revendication 1, dans laquelle ledit alkyle en C₁₋₆ est choisi dans le groupe consistant en méthyle, éthyle, n-propyle, isopropyle, n-butyle, isobutyle, cyclohéxyle, et n-pentyle.

4. Utilisation selon la revendication 1, dans laquelle ledit composé est choisi dans le groupe consistant en : 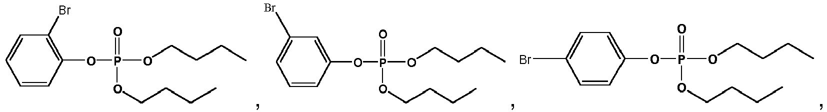 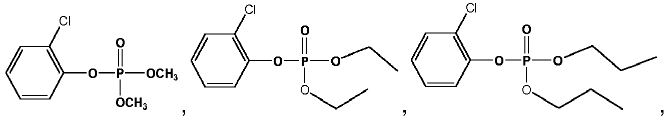 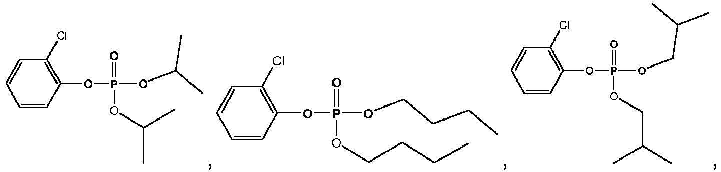 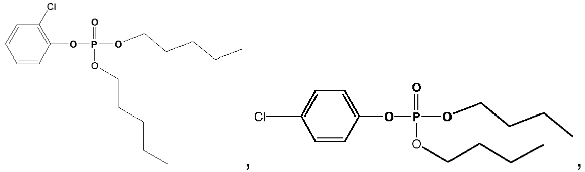 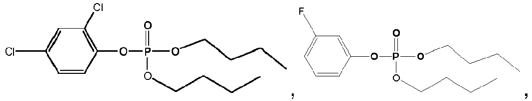 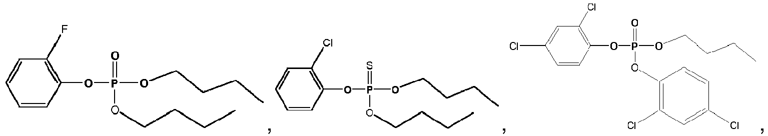 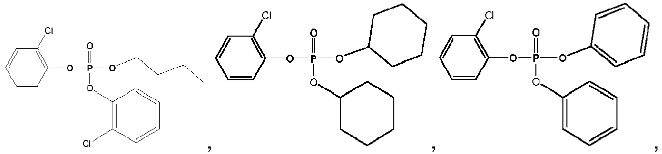 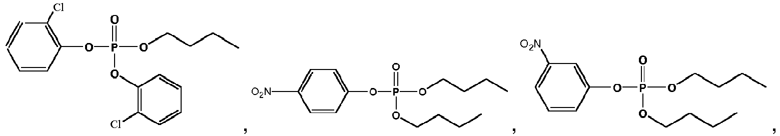 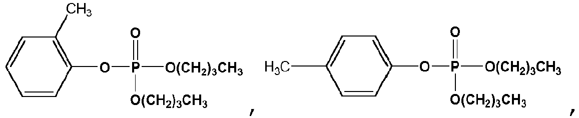 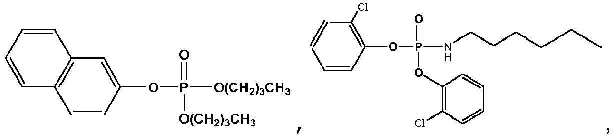 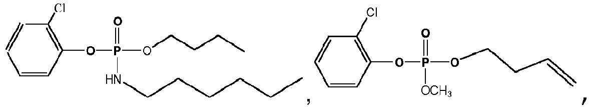 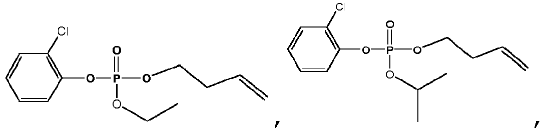 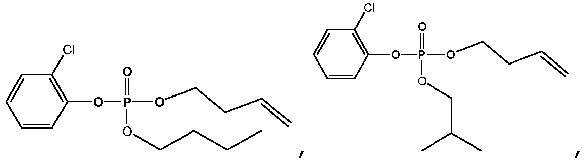 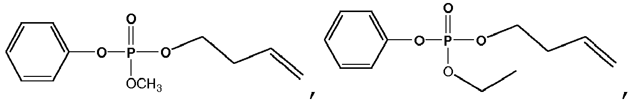 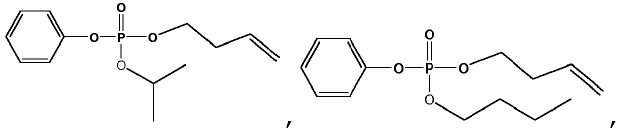 et

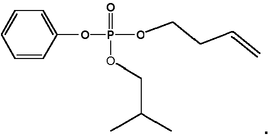

5. Utilisation d'un composé dans la fabrication d'un médicament pour le traitement d'une maladie neurodégénérative, dans laquelle ledit composé a la structure de Formules 1 et 2 : 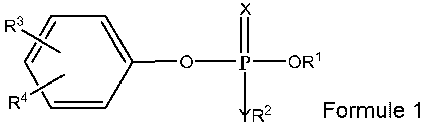 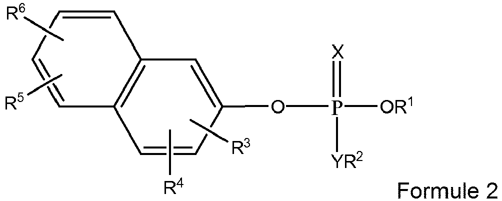 dans laquelle
X est O ou S ;
Y est O ou N ;
R¹ et R² peuvent être identiques ou différents et sont indépendamment choisis dans le groupe consistant en H, alkyle en C₁₋₆, alcényle en C₁₋₆, et phényle non substitué ou substitué ;
dans laquelle les substituants potentiels du groupe de phényle substitué sont choisis parmi F, Cl, Br, I, ou NO₂ ;
R³ et R⁴ en formule I peuvent être identiques ou différents et sont indépendamment choisis dans le groupe consistant en H, méthyle, méthoxy, F, Cl, Br et I ; et
R³ à R⁶ en formule II peuvent être identiques ou différents et sont indépendamment choisis dans le groupe consistant en H, méthyle, méthoxy, F, Cl, Br, I, et NO₂;
ou un sel pharmaceutiquement acceptable de celui-ci ; en inhibant le butyrylcholinestérase.

6. Utilisation selon la revendication 5, dans laquelle ledit phényle est substitué au moins une fois dans laquelle chaque substituant est indépendamment choisi dans le groupe consistant en H, méthyle, méthoxy, F, Cl, Br ou I en formule I ; et ledit phényle est substitué au moins une fois dans laquelle chaque substituant est indépendamment choisi dans le groupe consistant en H, méthyle, méthoxy, F, Cl, Br, I et NO₂ en formule II.

7. Utilisation selon la revendication 5, dans laquelle ledit alkyle en C₁₋₆ est choisi dans le groupe consistant en méthyle, éthyle, n-propyle, isopropyle, n-butyle, isobutyle, cyclohéxyle, et n-pentyle.

8. Utilisation selon la revendication 5, dans laquelle ledit composé est choisi dans le groupe consistant en : 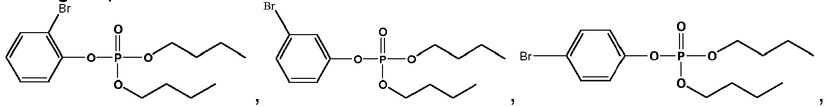 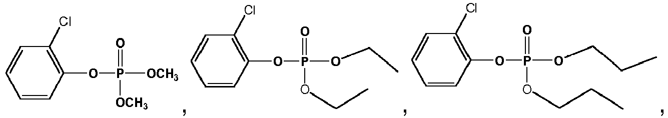 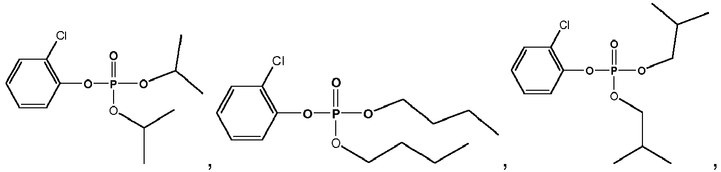 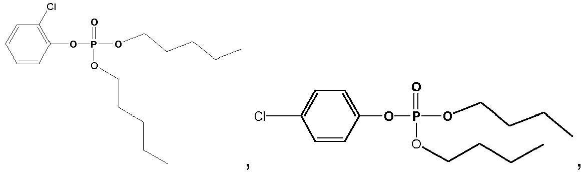 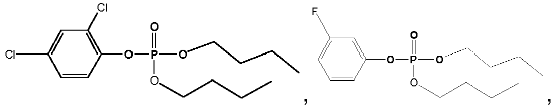 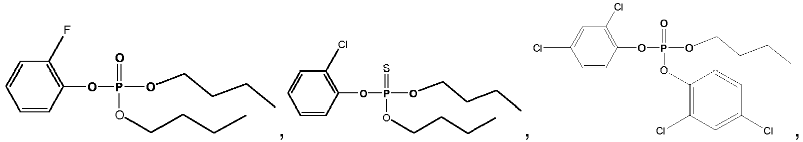 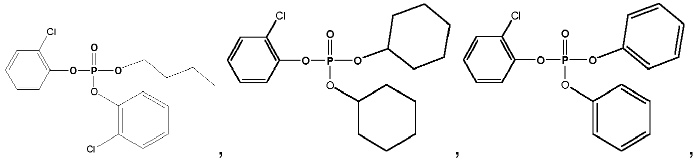 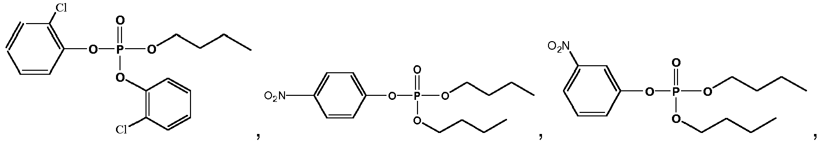 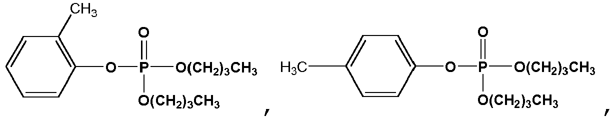 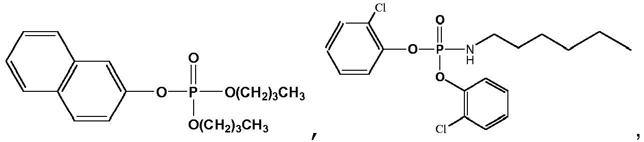 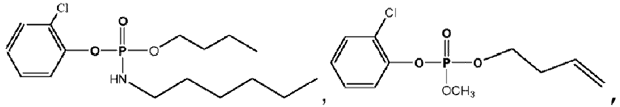 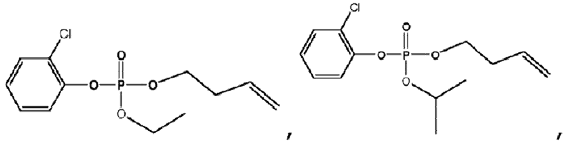 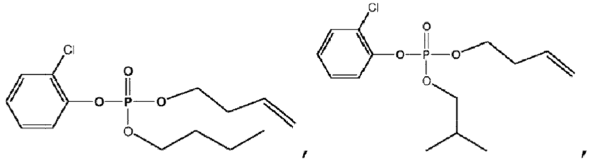 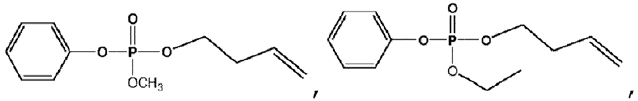 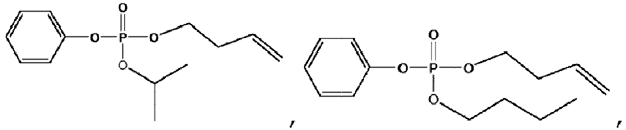 et

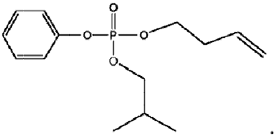

9. Utilisation selon la revendication 5 dans laquelle ladite maladie neurodégénérative est la maladie d'Alzheimer.
